# EUROPÄISCHE PATENTANMELDUNG

(11) **EP 4 664 477 A1**
(43) Veröffentlichungstag der Anmeldung: **17.12.2025**
(21) Anmeldenummer: 24181909.3
(22) Anmeldetag: 13.06.2024
(51) Int. Cl.: G16H 40/63, G16H 40/40, A61B 6/00, B25J 9/16, G06N 3/092

(54) **TECHNIK ZUR DYNAMISCHEN KOLLISIONSVERMEIDUNG FÜR EIN MEDIZINTECHNISCHES GERÄT**

(71) Anmelder: Siemens Healthineers AG, 91301 Forchheim (DE)
(72) Erfinder: Deinlein, Andreas, 95447 Bayreuth (DE); Scherlin, Erik, 95444 Bayreuth (DE)
(74) Vertreter: Siemens Healthineers Patent Attorneys

(57) **Zusammenfassung**

Die Erfindung betrifft eine Technik zum Trainieren eines dynamischen Kollisionsmodells für ein mobiles medizintechnisches Gerät. Ein computer-implementiertes Verfahren (100) umfasst ein Erfassen (S102) eines Hinweises auf eine medizintechnische Einsatzart (506) des Geräts. Eine Umgebungskarte (502) eines der Einsatzart (506) zugeordneten Einsatzorts des Geräts wird erfasst (S104). Ein dynamische Kollisionsmodell (516) für das Gerät wird mittels maschinellen Lernens (510) trainiert (S106). Das Trainieren (S106) basiert auf der Kombination des erfassten (S102) Hinweises auf die Einsatzart (506) und die erfasste (S104) Umgebungskarte (502) mit dem Einsatzort. Das Trainieren (S106) wird in einer Planungsphase und/oder Installationsphase des Geräts ausgeführt.

## Beschreibung

Es wird eine Technik zum Trainieren eines dynamischen Kollisionsmodells für ein mobiles medizintechnisches Gerät und zum Steuern einer Bewegung eines mobilen medizintechnischen Geräts anhand eines dynamischen Kollisionsmodells bereitgestellt. Die Technik umfasst insbesondere zwei Verfahren, eine Trainings-Vorrichtung, einen Controller, ein mobiles medizintechnisches Gerät, ein Computerprogrammprodukt und ein computerlesbares Speichermedium.

Kollisionsvermeidung ist ein wichtiges Feature bei medizinischen Geräten mit automatischen motorischen Bewegungsfunktionen. Für die Kollisionsvermeidung wird konventionellerweise ein Software (SW)-Algorithmus (oder "Kollisionsrechner") verwendet, welcher verhindern soll, dass Geräte untereinander, Geräte mit Equipment oder Raumelementen und Geräte mit Personen kollidieren. Der konventionelle Kollisionsvermeidungsalgorithmus arbeitet auf Basis von (insbesondere manuell gezeichneten primitiven) 3D Hüllen-Modellen von den Geräten und Equipment, welche herkömmlicherweise während einer Produktentwicklung vom Entwickler statisch erstellt werden. Die konventionellen 3D Hüllen-Modelle und SW-Algorithmen sind jedoch fehleranfällig und nicht optimal.

Die Verwendung von statischen Kollisionsmodellen in medizinischen Geräten mit motorischen Automatikfunktionen kann in einer Vielzahl von Szenarien zu Problemen führen, da der Algorithmus in Kombination mit dem Modell sich nicht an veränderliche und unvorhergesehene Umgebungsbedingungen anpassen kann. Das statische Modell wird im Voraus entwickelt und kann dabei nicht auf spontane Änderungen Rücksicht nehmen. Speziell problematisch ist das statische Modell in Bezug auf sich ändernde Aufnahmesituation und variierende Raumsituationen.

Wenn sich die Aufnahmesituation (etwa eine Tischaufnahme gegenüber einer freien Aufnahme) ändert - beispielsweise durch eine andere Positionierung des Patienten oder durch die Verwendung von zusätzlichem Equipment -, kann das Gerät herkömmlicherweise nicht intuitiv darauf reagieren, was zu ineffizienten Bewegungsabläufen oder im schlimmsten Fall zu Kollisionen führen kann. Dies kann nicht nur das Gerät beschädigen, sondern auch den Patienten gefährden.

Die Raumsituation in medizinischen Einrichtungen kann variieren. Ein Gerät, das herkömmlicherweise in einem definierten Raum entwickelt wurde, kann in einem Behandlungsraum oder in einem anderen klinischen Umfeld durch das räumliche Umfeld eingeschränkt sein. Ein statisches Kollisionsmodell kann nicht die spezifischen Gegebenheiten jedes Raumes berücksichtigen, was wiederum zu ineffizienten Bewegungen oder Kollisionen führen kann.

Die Problematiken des statischen Kollisionsmodell von medizinischen Geräten mit motorischen Automatikfunktionen werden herkömmlicherweise auf verschiedene Weisen angegangen, die eine manuelle Anpassung während der Installation, ein Umschalten voreingestellter Modelle, implementierte Sicherheitsmerkmale, regelmäßige Wartung und Updates sowie Benutzertraining umfassen.

Bei der manuellen Anpassung während der Installation werden herkömmliche Kollisionsmodelle während der Installation des Gerätes manuell an die spezifische Raumsituation angepasst. Servicetechniker passen vor Ort basierend auf der Raumgeometrie, den vorhandenen Hindernissen und anderen relevanten Faktoren die Konfiguration an. Basierend auf den eingestellten Randbedingungen wird dadurch das statische Kollisionsmodell, in den zur Verfügung stehenden Freiheitsgraden, angepasst. Dies ist fehleranfällig, da der Techniker die Objekte im Raum manuell ausmessen muss und in einer Service-Benutzerschnittstelle (fachsprachlich: User Interface, Ul) als Hindernisse eintragen muss.

Bei der herkömmlichen Umschaltung voreingestellter Modelle wird eine Variation an voreingestellten statischen Modellen, welche entsprechend der Konfiguration durch den Servicetechniker sowie entsprechend der aktuellen Applikation umschalten. Hierbei wird in spezifischen Anwendungsfällen bewusst das statische Kollisionsmodell mit einem anderen statischen Modell getauscht, um auf die Gegebenheiten entsprechend zu reagieren.

Die herkömmlicherweise implementierten Sicherheitsmerkmale dienen einer zusätzlichen Kollisionsminimierung durch das Integrieren von weiteren Sicherheitsmerkmalen, wie beispielsweise eine Not-Aus-Kreis, einer Geschwindigkeitsbegrenzung, einer elektronischen Abschaltung mittels Endschalter, einem mechanischer Endanschlag oder einer physikalischen Barriere.

Die regelmäßige Wartung und Updates führen herkömmlicherweise zu einer Anpassung der Systemkonfiguration durch den vor-Ort-Einsatz des Servicetechnikers. Beispielsweise kann der Servicetechniker ein Raumkonfiguration bei einer Wartung vor Ort ändern.

Eine Anpassung des statischen Kollisionsmodells an die Aufnahmesituation ist herkömmlicherweise meist nicht möglich, da dies zu dynamisch wäre. Das konventionelle Benutzertraining umfasst stattdessen Schulungen der Anwender bezüglich der Grenzen der einzelnen Geräte, sowie eventuell notwendige manuelle Eingriffe, um Kollisionen in den verschiedenen Aufnahmesituationen zu vermeiden. Hierfür sind erfahrene Benutzer notwendig, die wissen, wie sich das Gerät in bestimmten Situationen bewegt, um eine Kollision proaktiv zu vermeiden. Die Existenz solcher geeigneter erfahrener Benutzer (auch: Bediener) kann jedoch nicht mehr in der Breite erwartet werden.

Es ist daher ein Ziel der vorliegenden Erfindung, eine Lösung für eine verbesserte Kollisionsvermeidung und eine effizientere zumindest teil-automatisierte Bewegung eines mobilen medizintechnischen Geräts bereitzustellen. Alternativ oder ergänzend besteht die Aufgabe, eine dynamische Anpassung an zeitlich veränderliche medizinische Einsatzarten, Einsatzorte und bewegliche Hindernisse eines Kollisionsmodells zu ermöglichen, das insbesondere im medizinischen Gerät implementierbar ist.

Diese Aufgabe wird gelöst durch ein Verfahren zum Trainieren eines dynamischen Kollisionsmodells für ein mobiles medizintechnisches Gerät, durch ein Verfahren zum Steuern einer Bewegung eines mobilen medizintechnischen Geräts anhand eines dynamischen Kollisionsmodells, durch eine Trainings-Vorrichtung, durch einen Controller, durch ein mobiles medizintechnisches Gerät umfassend einen Controller, durch ein Computerprogramm (bzw. ein Computerprogrammprodukt) und durch ein computerlesbares Speichermedium gemäß den beigefügten unabhängigen Ansprüchen. Vorteilhafte Aspekte, Merkmale und Ausführungsformen sind in den abhängigen Ansprüchen und in der folgenden Beschreibung zusammen mit Vorteilen beschrieben.

Im Folgenden wird die erfindungsgemäße Lösung in Bezug auf die beanspruchten Verfahren zum Trainieren eines dynamischen Kollisionsmodells für ein mobiles medizintechnisches Gerät und zum Steuern einer Bewegung eines mobilen medizintechnischen Geräts anhand eines dynamischen Kollisionsmodells sowie in Bezug auf die beanspruchte Trainings-Vorrichtung und den beanspruchten Controller beschrieben. Merkmale, Vorteile oder alternative Ausführungsformen hierin können den anderen beanspruchten Gegenständen (z.B. dem mobilen medizintechnischen Gerät, dem Computerprogramm oder einem Computerprogrammprodukt) zugeordnet werden und umgekehrt. Mit anderen Worten: Die Ansprüche für die Trainings-Vorrichtung, den Controller und das mobile medizintechnische Gerät können durch Merkmale verbessert werden, die im Zusammenhang mit den Verfahren beschrieben oder beansprucht werden. In diesem Fall werden die funktionalen Merkmale des Verfahrens durch Struktureinheiten des Systems verkörpert und umgekehrt.

Gemäß einem ersten Verfahrensaspekt wird ein (insbesondere computer-implementiertes) Verfahren zum Trainieren eines dynamischen Kollisionsmodells für ein mobiles medizintechnisches Gerät bereitgestellt. Das Verfahren umfasst einen Schritt des Erfassens eines Hinweises auf eine medizintechnische Einsatzart eines mobilen medizintechnischen Geräts. Das Verfahren umfasst ferner einen Schritt des Erfassens einer Umgebungskarte eines Einsatzorts des mobilen medizintechnischen Geräts, der der medizintechnischen Einsatzart zugeordnet ist. Das Verfahren umfasst ferner einen Schritt des Trainierens eines dynamischen Kollisionsmodells für das mobile medizintechnische Gerät. Das Trainieren erfolgt mittels maschinellen Lernens (ML) und basiert auf der Kombination des erfassten Hinweises auf die Einsatzart und die erfasste Umgebungskarte mit dem Einsatzort. Das Trainieren (mittels ML) des dynamischen Kollisionsmodells wird in einer Planungsphase und/oder Installationsphase des mobilen medizintechnischen Geräts ausgeführt.

Mittels der erfindungsgemäßen Technik wird eine zuverlässige Kollisionsvermeidung bei der mechatronischen Bewegung eines mobilen medizintechnischen Geräts (kurz auch: medizintechnisches Gerät oder medizinisches Gerät) ermöglicht. Alternativ oder ergänzend wird eine effiziente Bestimmung einer (insbesondere mechatronischen) Bewegung des medizinischen Geräts ermöglicht. Die Effizienz kann sich auf eine Zeit-Effizienz, eine Effizienz im Rechenaufwand (z.B. hinsichtlich eines auf dem medizinischen Gerät angeordneten Prozessors, der in einer Anwendungsphase das dynamische Kollisionsmodell auswertet) und/oder eine Effizienz hinsichtlich eines Speicherplatzes (z.B. für das trainierte dynamische Kollisionsmodell) umfassen.

Die Zeit-Effizienz kann beispielsweise eine Steuerzykluszeit der Bewegung des mobilen medizintechnischen Geräts (z.B. 50ms) berücksichtigen.

Weiterhin alternativ oder ergänzend wird durch die erfindungsgemäße Technik eine effiziente Bewegung des medizinischen Geräts ermöglicht, bei der insbesondere gleichzeitig Verzögerungen und Beschädigungen am Gerät vermieden werden sowie eine Sicherheit eines Patienten berücksichtigt wird, der mittels des medizinischen Geräts therapiert oder diagnostiziert wird. Alternativ oder ergänzend werden Verletzungen vermieden, sowohl für den Patienten als auch für medizinisches Personal, das sich in der Umgebung des medizinischen Geräts befindet. Beispielsweise kann das medizinische Personal das mobile medizintechnische Gerät für die Einsatzart bedienen (z.B. für eine Aufnahme eines Bilddatensatzes programmieren).

Mittels der erfindungsgemäßen Technik ist ferner eine Optimierung und Validierung möglich für das dynamische Kollisionsmodell. Das Kollisionsmodells kann beispielsweise bereits in der Planungsphase des medizinischen Geräts erstellt und/oder trainiert werden. Hierzu kann beispielsweise ein digitaler Zwilling und/oder ein mechatronisches Bewegungsmodell (z.B. ein CAD-Modell) des medizinischen Geräts verwendet werden. Eine Validierung kann in der Installationsphase erfolgen. Hierzu können Sensoren verwendet werden, die mögliche Kollisionen registrieren. Die zugehörigen Sensordaten können mit Ergebnissen des trainierten dynamischen Kollisionsmodells verglichen werden.

Die medizintechnische Einsatzart (auch: medizinische Einsatzart, medizinischer Anwendungsfall bzw. medizinische Applikation, kurz: medizinische Anwendung) kann eine Aufnahme von medizinischen Bilddaten umfassen. Beispielsweise kann das mobile medizintechnische Gerät eine Patientenliege (auch: Patiententisch) für ein medizinisches bildgebendes Gerät umfassen (z.B. für einen Magnetresonanztomographen, MRT). Alternativ oder ergänzend kann das mobile medizintechnische Gerät ein mobiles medizinisches bildgebendes Gerät (oder zumindest eine Komponente davon) umfassen, beispielsweise einen mobilen Computertomographen (CT), ein mobiles Röntgengerät oder einen C-Bogen. An dem C-Bogen können eine Quelle und ein Detektor eines CT oder eines Röntgengeräts angeordnet sein.

Das mobile medizintechnische Gerät (kurz auch: das medizinische Gerät bzw. das Gerät) kann eine Aktorik umfassen, um sich in einer Ebene motorisch unterstützt fortzubewegen. Alternativ oder ergänzend kann das mobile medizintechnische Gerät eine Mehrzahl Rollen umfassen, auf denen es in einer Ebene beweglich sein kann. Beispielsweise kann je eine Rolle oder eine Achse mit Rollen eine zugeordnete Aktorik umfassen.

Die medizintechnische Einsatzart kann Informationen über Anforderungen und/oder Einschränkungen an die Bewegungen des mobilen medizintechnischen Geräts bereitstellen (und/oder umfassen), beispielsweise hinsichtlich eines Raumbedarfs für medizinisches Personal, weitere medizintechnische Geräte (z.B. ein Gerät zur Überwachung von Vitalfunktionen, beispielsweise in einem Operationssaal, und/oder ein Beistelltisch mit sterilisierten bzw. bereits benutzen Operationsbesteckteilen) und/oder Ausstattungsgegenstände für die Einsatzart (z.B. eine Armhalterung und/oder Spulen für einen MRT). Alternativ oder ergänzend kann die medizintechnische Einsatzart Informationen über eine geplante Positionierung (auch: Lage) des Patienten umfassen (z.B. eine Seitenlage auf der Patientenliege). Die geplante Positionierung des Patienten kann beispielsweise zu einem beweglichen (auch: mobilen) Hindernis führen, z.B. wenn ein Arm oder andere Gliedmaßen über die Patientenliege hinausragen in der geplanten Positionierung.

Die Umgebungskarte (auch: Lageplan und/oder Grundriss) kann eine medizinische Einrichtung oder einen Bereich innerhalb einer medizinischen Einrichtung umfassen, z.B. die Räume einer Radiologie-Abteilung. Die Umgebungskarte kann einen Grundriss einer befahrbaren Bodenfläche umfassen (beispielsweise mit bereits berücksichtigten nicht-beweglichen Hindernissen wie fest montierten Einrichtungsgegenständen oder weiteren medizintechnischen Geräten). Zusätzlich kann die Umgebungskarte Bereiche umfassen, die nur bis zu einer vorbestimmten Höhe unterfahrbar sind (z.B. ein auf Beinen stehender Tisch).

Das dynamische Kollisionsmodell (auch: ML-Modell) kann darauf trainiert sein, mobile Hindernisse zu berücksichtigen. Ein mobiles Hindernis kann z.B. eine Person sein. Beispielsweise kann medizinisches Personal typische Ortsänderungen ausführen während eines Einsatzes des mobilen medizintechnischen Geräts. Alternativ oder ergänzend kann ein Patient umgelagert werden und/oder dazu aufgefordert werden, seine Position zu verändern, insbesondere abhängig von der Einsatzart. Während der Entwicklungsphase können Veränderungen der mobilen Hindernisse berücksichtigt werden.

Das Training des dynamischen Kollisionsmodells kann anhand von synthetischen Daten erfolgen, beispielsweise basierend auf Simulationen von Abläufen medizinischer Einsätze. In den Simulationen können Ortsänderungen und/oder Bewegungsabläufe von Personen berücksichtigt werden.

Der medizinische Einsatz kann eine Behandlung und/oder eine Diagnose sein. Der medizinische Einsatz kann insbesondere die medizintechnische Einsatzart des mobilen medizintechnischen Geräts umfassen.

Alternativ oder ergänzend kann das Training des dynamischen Kollisionsmodells anhand von realen Daten erfolgen. Beispielsweise können reale Daten Bewegungsabläufe bei einem bereits im Einsatz befindlichen mobilen medizintechnischen Gerät (insbesondere für die gleiche Einsatzart und/oder mit ähnlichem Bautyp) umfassen.

In der Trainingsphase können synthetische Datensätze und reale Datensätze verwendet werden. Die Verwendung von synthetischen Datensätzen ermöglicht insbesondere ein Training von seltenen Fällen (auch: Spezialfällen).

Für das Training kann ein erweiterter Bewegungsraum (auch: "Feature Space") des mobilen medizintechnischen Geräts verwendet werden.

Das mobile medizintechnische Gerät kann in seiner Hardware (und/oder seinen Baukomponenten) vollentwickelt sein.

Die Planungsphase kann sich auf eine Entwicklung von lokal am mobilen medizintechnischen Gerät gespeicherter Software (z.B. Steuerungs-Software und/oder Regelungs-Software) beziehen. Insbesondere kann in der Planungsphase bereits bekannt sein, in welcher Umgebung das mobile medizintechnische Gerät eingesetzt werden soll. Alternativ oder ergänzend kann eine Umgebungskarte aller möglichen Einsatzorte bekannt sein.

Die Installationsphase kann umfassen, dass das mobile medizinische Gerät gemäß entwickeltem Plan hergestellt wird. Alternativ oder ergänzend kann die Installationsphase umfassen, dass das mobile medizinische Gerät in der Umgebung der möglichen Einsatzorte positioniert wird. Ferner kann die Installationsphase Funktionstests des mobilen medizinischen Geräts umfassen, beispielsweise beim Hersteller und/oder nach Positionierung in der Umgebung der möglichen Einsatzorte.

Das dynamische Kollisionsmodell kann zunächst vortrainiert sein, beispielsweise zunächst statisch lediglich anhand von einer oder mehreren Umgebungskarten.

Das dynamische Kollisionsmodell kann anhand von einer Mehrzahl von Umgebungskarten trainiert sein. Die Mehrzahl von Umgebungskarten kann disjunkt (und/oder nicht verbunden) sein. Beispielsweise kann das Training in der Planungsphase für eine Vielzahl von medizinischen Einrichtungen erfolgen.

Die medizinischen Einrichtungen können zu einem (z.B. Krankenhaus-) Verbund gehören. Alternativ oder ergänzend kann das mobile medizintechnische Gerät basierend auf einer Vielzahl von Umgebungskarten unterschiedlicher Kunden beim Hersteller trainiert werden.

Das Training kann ein Transfer-Lernen (fachsprachlich: Transfer Learning) umfassen. Beispielsweise kann das Training anhand von Umgebungskarten erfolgen, die nicht der Umgebungskarte der geplanten Einsatzorte entspricht. Alternativ oder ergänzend kann das Transfer-Lernen umfassen, dass das Training für ähnliche (insbesondere nicht identische) medizintechnische Einsatzarten erfolgt, für die das mobile medizintechnische Gerät im späteren Einsatz vorgesehen ist.

Das Vortraining und/oder Training des dynamischen Kollisionsmodells kann für eine Vielzahl von mobilen medizintechnischen Geräten gleichen Bautyps simultan ausgeführt werden. Das Vortraining und/oder Training kann beim Hersteller und/oder auf einer Trainings-Vorrichtung erfolgen. Die (z.B. zentrale) Trainings-Vorrichtung kann beispielsweise eine große Rechenkapazität und/oder einen großen Speicher haben.

Das dynamische Kollisionsmodell kann später nachtrainiert werden, insbesondere speziell für eine Umgebung, in der das mobile medizintechnische Gerät positioniert worden ist. Das Nachtraining kann auf einem Controller (auch: Steuerung und Regelung) des mobilen medizintechnischen Geräts (insbesondere lokal) ausgeführt werden. Der Controller kann eine kleine Rechenkapazität und/einen kleinen Speicher haben, insbesondere im Vergleich zu der (z.B. zentralen) Trainings-Vorrichtung.

Sie medizintechnische Einsatzart kann eine Aufnahme von medizinischen Bilddaten sein. Alternativ oder ergänzend kann das mobile medizintechnische Gerät ein mobiles bildgebendes Gerät oder eine Komponente eines mobilen bildgebenden Gerätes sein. Optional kann die Komponente ein C-Bogen oder ein Patiententisch sein.

Das mobile medizintechnische Gerät kann ein CT oder ein Röntgengerät sein. Alternativ kann das mobile medizintechnische Gerät eine Patientenliege (auch: Patiententisch) für einen MRT sein.

Mittels des trainierten dynamischen Kollisionsmodells kann in einer Anwendungsphase beispielsweise vermieden werden, dass der Patient bei einer Aufnahme verletzt wird.

Das Verfahren kann ferner einen Schritt des Vortrainieren des dynamische Kollisionsmodells umfassen. Das Vortrainieren kann insbesondere statisch sein und/oder anhand mindestens einer Umgebungskarte.

Alternativ oder ergänzend kann das Verfahren einen Schritt des Erfassens von Positionsdaten hinsichtlich eines mobilen Hindernisses im Bereich der Umgebungskarte umfassen. Alternativ oder ergänzend kann das Verfahren einen Schritt des Erfassens des Einsatzorts des mobilen medizintechnischen Geräts umfassen.

Weiterhin alternativ oder ergänzend kann das Verfahren einen Schritt des Nachtrainieren des dynamischen Kollisionsmodells umfassen. Insbesondere kann das Nachtrainieren in einer Einsatzphase des mobilen medizintechnischen Geräts erfolgend basierend auf realen historischen Bewegungsdaten. Die Bewegungsdaten können Kollisionsdaten umfassen.

Das Vortrainieren kann (insbesondere zentral) für eine Vielzahl mobiler medizinscher Geräte erfolgen, beispielsweise für unterschiedliche Einsatzarten. Das Vortrainieren kann beispielsweise agnostisch bezüglich der medizintechnischen Einsatzart sein.

Das Erfassen von Positionsdaten kann ein Erfassen von (insbesondere synthetischen und/oder realen) Sensordaten hinsichtlich des mobilen Hindernisses umfassen. Beispielsweise können Bewegungen von Personen simuliert werden. Die erfassten Positionsdaten können beim Training des dynamischen Kollisionsmodells (insbesondere zusätzlich zu der Einsatzart und der Umgebungskarte) berücksichtigt werden.

Anhand der Umgebungskarte und den erfassten Positionsdaten kann ein Umgebungsmodell des mobilen medizintechnischen Geräts erstellt werden. Das Umgebungsmodell kann beispielsweise einen Agenten pro mobilem Hindernis sowie einen Agenten für das mobile medizintechnische Gerät umfassen. Das dynamische Kollisionsmodell kann auf dem Umgebungsmodell basieren.

Das Nachtrainieren kann lokal auf dem mobilen medizintechnischen Gerät erfolgen. Alternativ oder ergänzend ist ein Nachtrainieren in einer Cloud und/oder auf einer zentralen Rechenvorrichtung (auch: Computer) der medizinischen Einrichtung möglich, in der das mobile medizintechnische Gerät eingesetzt wird.

Das Verfahren kann in der Installationsphase ferner einen Schritt des Erfassens von Umgebungssensordaten hinsichtlich des Einsatzortes während Testbewegungen des mobilen medizintechnischen Geräts umfassen. Alternativ oder ergänzend kann das Verfahren in der Installationsphase ferner einen Schritt des Validierens des trainierten dynamischen Kollisionsmodells umfassen. Das Validieren kann ein Abgleichen der erfassten Umgebungssensordaten mit einem Zustand gemäß dem trainierten dynamischen Kollisionsmodell umfassen.

Das Validieren kann ein Validieren eines trainierten Umgebungsmodells umfassen.

Mittels des Validierens kann sichergestellt werden, dass das dynamische Kollisionsmodell zuverlässig Kollisionen am Einsatzort vermeiden kann.

Das Validieren kann anhand von Umgebungssensordaten erfolgen, die mittels einer Sensoreinheit mit Sensoren am mobilen medizintechnischen Gerät und/oder mit Sensoren innerhalb der medizinischen Einrichtung, für die die Umgebungskarte umfassen die Einsatzorte, für die das dynamische Kollisionsmodell trainiert wurde, erfasst wurden.

Das Validieren kann in einer Cloud, auf einer zentralen Rechenvorrichtung (auch: Computer) der medizinischen Einrichtung erfolgen und/oder lokal auf dem mobilen medizintechnischen Gerät.

Beim Trainieren (mittels ML) des dynamischen Kollisionsmodells kann mindestens eine Randbedingung und/oder Zusatzbedingung berücksichtigt werden. Optional kann die Randbedingung und/oder Zusatzbedingung eine vorbestimmte Bedingung an einen Bewegungsablauf umfassen, insbesondere eine Geschwindigkeitsvorgabe. Alternativ oder ergänzend kann die Randbedingung und/oder Zusatzbedingung eine Benutzerunterstützung einer Bewegung umfassen.

Die Geschwindigkeitsvorgaben kann insbesondere eine maximale Geschwindigkeit umfassen. Die Geschwindigkeitsvorgabe kann individuell pro Raumpunkt sein. Beispielsweise kann die maximale Geschwindigkeit höher sein für einen Korridorbereich, der üblicherweise frei von mobilen Hindernissen ist, als für einen Bereich nahe am Einsatzort, z.B. am Ort einer Aufnahme von medizinischen Bilddaten. Beispielsweise können in dem Bereich nahe am Einsatzort üblicherweise mobile Hindernisse vorhanden sein, z.B. Personen, weitere mobile medizintechnische Geräte und/oder Beistelltische. Hierdurch kann ein möglichst sicherer Bewegungsablauf bei gleichzeitiger Zeiteffizienz ermöglicht werden.

Die Benutzerunterstützung der Bewegung kann umfassen, dass das mobile medizintechnische Gerät sich (z.B. zeitweise) nicht vollkommen autonom bewegt. Beispielsweise kann eine Benutzerunterstützung der der Bewegung für einen Bereich mit vielen mobilen Hindernissen geeignet sein, z.B. für den Bereich nahe am Einsatzort. Hierdurch kann eine Kollisionsfreiheit zeiteffizient sichergestellt werden.

Die Randbedingung und/oder Zusatzbedingung kann alternativ oder ergänzend Sicherheitsmerkmale berücksichtigen, beispielsweise einen mechanischen Endanschlag und/oder ein Not-Aus bei einer Kollision mit einer physikalischen Barriere.

Das ML kann ein verstärkendes Lernen (RL) verwenden. Alternativ oder ergänzend kann das Trainieren des Kollisionsmodells einen digitalen Zwilling des mobilen medizintechnischen Geräts verwenden.

Das ML kann ein mechatronisches Bewegungsmodell des mobilen medizintechnischen Geräts (z.B. ein CAD-Modell) verwenden. Beispielsweise kann ein digitaler Zwilling eine visuelle Hülle und System Control Software umfassen. Mittels des digitalen Zwillings kann Feedback zu Kollisionen mechatronisch beweglicher Elemente trainiert werden (z.B. anhand von synthetischen Trainingsdaten).

Das RL kann einen Agenten für das mobile medizintechnische Gerät sowie einen Agenten pro mobiles Hindernis umfassen.

Das RL kann mindestens eine Strategie (fachsprachlich: Policy) und/oder mindestens eine Belohnung (fachspachlich: Reward) umfassen, um das Training (und/oder Lernverhalten) des dynamischen Kollisionsmodells zu lenken. Beispielsweise können ein aktueller Bewegungszustand (insbesondere eine Position und/oder Orientierung) des mobilen medizintechnischen Geräts sowie die aktuelle Umgebung, beispielsweise zusammengefasst in einem Umgebungsmodell, berücksichtigt werden. Alternativ oder ergänzend kann ein Hyperparameter-Tuning des dynamischen Kollisionsmodells anhand einer Value Function, einer Discount Function, einer Learning Rate und/oder von Exploration x Exploitation ausgeführt werden.

Zumindest der Schritt des Trainierens des dynamischen Kollisionsmodells kann außerhalb des mobilen medizintechnischen Geräts ausgeführt werden.

Das Trainieren kann auf einer zentralen Rechenvorrichtung und/oder auf einem Edge Device (z.B. beim Hersteller oder in der medizinischen Einrichtung, in der das mobile medizintechnische Gerät nach der Installationsphase eingesetzt werden soll) ausgeführt werden. Dadurch kann für das Training ein große Prozessorkapazität und/oder Speicherkapazität (z.B. zeitlich begrenzt) bereitgestellt werden.

Das trainierte dynamische Kollisionsmodell kann lokal auf dem mobilen medizintechnischen Gerät gespeichert und ausgeführt werden.

Das trainierte dynamische Kollisionsmodell kann auf einem Prozessor mit geringer Kapazität ausführbar sein. Alternativ oder ergänzend kann das trainierte dynamische Kollisionsmodell in einem Speicher mit geringer Kapazität ausführbar sein.

Gemäß einem zweiten Verfahrensaspekt wird ein (insbesondere computer-implementiertes) Verfahren zum Steuern einer Bewegung eines mobilen medizintechnischen Geräts anhand eines dynamischen Kollisionsmodells bereitgestellt. Das Verfahren umfasst einen Schritt des Empfangens eines Hinweises auf eine medizinische Einsatzart und einen zugeordneten Einsatzort eines mobilen medizintechnischen Geräts. Das Verfahren umfasst ferner einen Schritt des Erfassens von Umgebungssensordaten des mobilen medizintechnischen Geräts. Das Verfahren umfasst ferner einen Schritt des Auswertens der erfassten Umgebungssensordaten und Bestimmen, mittels eines gemäß dem ersten Verfahrensaspekt trainierten dynamischen Kollisionsmodells, eines Steuersignals zum kollisionsfreien Steuern des mobilen medizintechnischen Geräts.

Das Verfahren umfasst ferner einen Schritt des Bereitstellens des bestimmten Steuersignals an eine Bewegungsaktorik des mobilen medizintechnischen Geräts.

Der Hinweis auf den Einsatzort kann implizit sein. Insbesondere kann durch den Hinweis auf die medizinische Einsatzart der zugeordnete Einsatzort festgelegt und/oder bekannt sein. Beispielsweise kann nach Installation des mobilen medizinischen Geräts der medizinischen Einsatzart einem vorbestimmten Einsatzort (z.B. immer) zugeordnet sein. Beispielsweise kann in einer medizinischen Einrichtung der Einsatzort für eine einem bildgebenden Gerät zugeordnete mobile Patientenliege fest vorbestimmt sein (z.B. aufgrund einer Einbausituation eines MRT).

Das Auswerten der Umgebungssensordaten kann ein Erstellen eines Umfeldmodells unter Verwendung der Umgebungskarte umfassen, mittels der das dynamische Kollisionsmodell trainiert wurde.

Die Umgebungssensordaten können mittels einer Sensoreinheit empfangen werden. Die Sensoreinheit kann Sensoren am mobilen medizintechnischen Gerät umfassen und/oder Sensoren außerhalb des medizintechnischen Geräts, beispielsweise in der medizinischen Einrichtung (z.B. Kameras). Die mittels außerhalb des medizintechnischen Geräts aufgenommenen Umgebungssensordaten können drahtlos an das mobile medizinische Gerät übertragen werden.

Gemäß einem ersten Vorrichtungsaspekt wird eine Trainings-Vorrichtung zum Trainieren eines dynamischen Kollisionsmodells für ein mobiles medizintechnisches Gerät bereitgestellt. Die Trainings-Vorrichtung umfasst eine Hinweis-Schnittstelle, die zum Erfassen eines Hinweises auf eine medizintechnische Einsatzart eines mobilen medizintechnischen Geräts ausgebildet ist. Die Trainings-Vorrichtung umfasst ferner eine Umgebungskarten-Schnittstelle, die zum Erfassen einer Umgebungskarte eines der medizintechnischen Einsatzart zugeordneten Einsatzorts des mobilen medizintechnischen Geräts ausgebildet ist. Die Trainings-Vorrichtung umfasst ferner eine Trainingseinheit, die zum Trainieren, mittels ML, eines dynamischen Kollisionsmodells für das mobile medizintechnische Gerät basierend auf der Kombination des erfassten Hinweises auf die Einsatzart und die erfasste Umgebungskarte mit dem Einsatzort ausgebildet ist. Das Trainieren (mittels ML) des dynamischen Kollisionsmodells ist in einer Planungsphase und/oder Installationsphase des mobilen medizintechnischen Geräts ausführbar.

Die Trainings-Vorrichtung kann ferner Merkmale umfassen, die im Kontext des ersten Verfahrensaspekts offenbart sind. Alternativ oder ergänzend kann die Trainings-Vorrichtung dazu ausgebildet sein, Verfahrensschritte gemäß dem ersten Verfahrensaspekt auszuführen.

Gemäß einem zweiten Vorrichtungsaspekt wird ein Controller zum Steuern einer Bewegung eines mobilen medizintechnischen Geräts anhand eines dynamischen Kollisionsmodells bereitgestellt. Der Controller umfasst eine Hinweis-Schnittstelle, die zum Empfangen eines Hinweises auf eine medizinische Einsatzart und einen zugeordneten Einsatzort eines mobilen medizintechnischen Geräts ausgebildet ist. Der Controller umfasst ferner eine Umgebungssensordaten-Schnittstelle, die zum Erfassen von Umgebungssensordaten des mobilen medizintechnischen Geräts ausgebildet ist. Der Controller umfasst ferner eine Auswerteeinheit, die zum Auswerten der erfassten Umgebungssensordaten ausgebildet ist. Der Controller umfasst eine Bestimmungseinheit, die zum Bestimmen, mittels eines gemäß dem ersten Verfahrensaspekt (und/oder durch Trainings-Vorrichtung des ersten Vorrichtungsaspekts) trainierten dynamischen Kollisionsmodells, eines Steuersignals zum kollisionsfreien Steuern des mobilen medizintechnischen Geräts ausgebildet ist. Der Controller umfasst ferner eine Steuersignal-Schnittstelle, die zum Bereitstellen des bestimmten Steuersignals an eine Bewegungsaktorik des mobilen medizintechnischen Geräts ausgebildet ist.

Der Controller kann zum Ausführen des Verfahrens gemäß dem zweiten Verfahrensaspekt ausgebildet sein.

Gemäß einem dritten Vorrichtungsaspekt wird ein mobiles medizintechnisches Gerät bereitgestellt. Das mobile medizintechnische Gerät umfasst einen Controller gemäß dem zweiten Vorrichtungsaspekt.

Gemäß einem weiteren Aspekt wird ein Computerprogrammprodukt mit Programmelementen bereitgestellt, die eine Trainings-Vorrichtung veranlassen, die Schritte des Verfahrens zum Trainieren eines dynamischen Kollisionsmodells für ein mobiles medizintechnisches Gerät gemäß dem ersten Verfahrensaspekt auszuführen, wenn die Programmelemente in einen Speicher der Trainings-Vorrichtung geladen werden. Alternativ oder ergänzend wird ein Computerprogrammprodukt mit Programmelementen bereitgestellt, die einen Controller veranlassen, die Schritte des Verfahrens zum Steuern einer Bewegung eines mobilen medizintechnischen Geräts anhand eines dynamischen Kollisionsmodells gemäß dem zweiten Verfahrensaspekt auszuführen, wenn die Programmelemente in den Speicher des Controllers geladen werden.

Gemäß einem noch weiteren Aspekt wird ein computerlesbares Speichermedium bereitgestellt, auf dem Programmelemente gespeichert sind, die von einer Trainings-Vorrichtung gelesen und ausgeführt werden können, um Schritte des Verfahrens zum Trainieren eines dynamischen Kollisionsmodells für ein mobiles medizintechnisches Gerät gemäß dem ersten Verfahrensaspekt durchzuführen, wenn die Programmelemente von der Trainings-Vorrichtung ausgeführt werden. Alternativ oder ergänzend wird ein computerlesbares Speichermedium bereitgestellt, auf dem Programmelemente gespeichert sind, die von einem Controller gelesen und ausgeführt werden, um Schritte des Verfahrens zum Steuern einer Bewegung eines mobilen medizintechnischen Geräts anhand eines dynamischen Kollisionsmodells gemäß dem zweiten Verfahrensaspekt durchzuführen, wenn die Programmelemente von dem Controller ausgeführt werden.

Die oben beschriebenen Eigenschaften, Merkmale und Vorteile der vorliegenden Erfindung sowie die Art und Weise, wie sie erreicht werden, werden im Lichte der folgenden Beschreibung und der Ausführungsbeispiele, die im Zusammenhang mit den Zeichnungen näher erläutert werden, klarer und verständlicher. Diese folgende Beschreibung beschränkt die Erfindung nicht auf die enthaltenen Ausführungsformen. Gleiche Komponenten oder Teile können in verschiedenen Figuren mit den gleichen Bezugszeichen versehen sein. Im Allgemeinen sind die Abbildungen nicht maßstabsgetreu.

Es versteht sich, dass eine bevorzugte Ausführungsform der vorliegenden Erfindung auch eine beliebige Kombination der abhängigen Ansprüche oder der obigen Ausführungsformen mit dem jeweiligen unabhängigen Anspruch sein kann.

Diese und andere Aspekte der Erfindung werden aus den nachfolgend beschriebenen Ausführungsformen ersichtlich und werden durch Bezugnahme auf diese erläutert.

### Kurze Beschreibung der Zeichnungen:

- Fig. 1: ist ein Flussdiagramm eines Verfahrens zum Trainieren eines dynamischen Kollisionsmodells für ein mobiles medizintechnisches Gerät gemäß einer bevorzugten Ausführungsform der vorliegenden Erfindung;
- Fig. 2: ist ein Flussdiagramm eines Verfahrens zum Steuern einer Bewegung eines mobilen medizintechnischen Geräts anhand eines dynamischen Kollisionsmodells gemäß einer bevorzugten Ausführungsform der vorliegenden Erfindung, wobei das dynamische Kollisionsmodell gemäß dem Verfahren der Fig. 1 trainiert sein kann;
- Fig. 3: ist eine Übersicht über die Struktur und den Aufbau einer Trainings-Vorrichtung zum Trainieren eines dynamischen Kollisionsmodells für ein mobiles medizintechnisches Gerät gemäß einer bevorzugten Ausführungsform der vorliegenden Erfindung;
- Fig. 4: ist eine Übersicht über die Struktur und den Aufbau eines Controllers zum Steuern einer Bewegung eines mobilen medizintechnischen Geräts anhand eines dynamischen Kollisionsmodells gemäß einer bevorzugten Ausführungsform der vorliegenden Erfindung;
- Fig. 5: zeigt schematisch ein Ausführungsbeispiel des Verfahrens zum Trainieren eines dynamischen Kollisionsmodells für ein mobiles medizintechnisches Gerät in einer Planungsphase und/oder Installationsphase, wobei das Trainings-Verfahren eine Variante des Verfahrens der Fig. 1 sein kann;
- Fig. 6: zeigt schematisch ein Ausführungsbeispiel des Verfahrens zum Steuern einer Bewegung eines mobilen medizintechnischen Geräts anhand eines dynamischen Kollisionsmodells in einer Inferenzphase, wobei das Bewegungssteuerungs-Verfahren eine Variante des Verfahrens der Fig. 2 sein kann;
- Fig. 7: zeigt ein erweitertes Flussdiagramm zum Erstellen und Einsetzen eines dynamischen Kollisionsmodells für ein mobiles medizintechnisches Gerät gemäß einer bevorzugten Ausführungsform der vorliegenden Erfindung;
- Fig. 8: schematisch ein verstärkendes Lernen mittels eines Agenten, Zuständen und Belohnungen sowie Strategien, wodurch ein dynamisches Kollisionsmodell beispielsweise optimiert werden kann.
- Fig. 9: zeigt ein weiteres erweitertes Flussdiagramm zum Erstellen und Einsetzen eines dynamischen Kollisionsmodells für ein mobiles medizintechnisches Gerät gemäß einer bevorzugten Ausführungsform der vorliegenden Erfindung.

Etwaige Bezugszeichen in den Ansprüchen sind nicht als Einschränkung des Anwendungsbereichs zu verstehen.

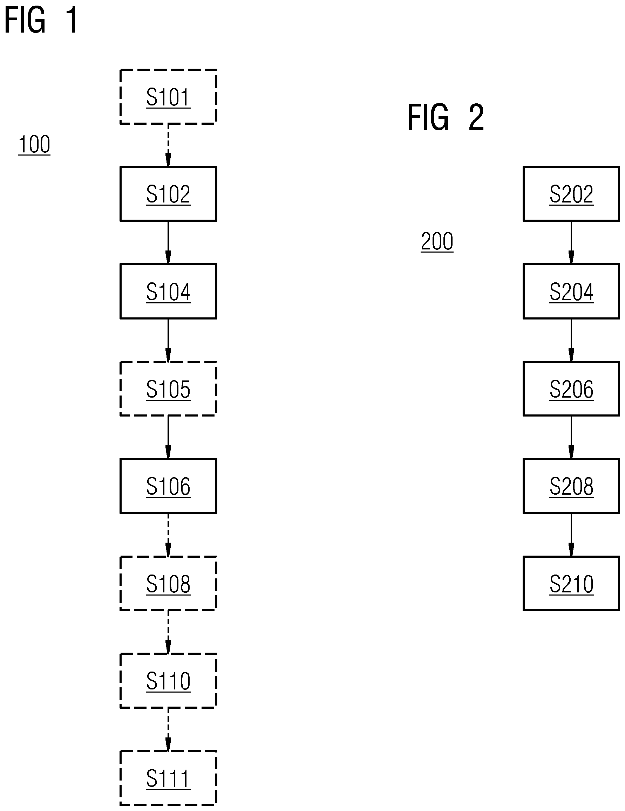

Fig. 1 zeigt schematisch ein beispielhaftes Ablaufdiagramm eines (insbesondere computer-implementierten) Verfahrens 100 zum Trainieren eines dynamischen Kollisionsmodells für ein mobiles medizintechnisches Gerät.

Das Verfahren 100 umfasst einen Schritt S102 des Erfassens eines Hinweises auf eine medizintechnische Einsatzart eines mobilen medizintechnischen Geräts. Das Verfahren 100 umfasst ferner einen Schritt S104 des Erfassens einer Umgebungskarte eines der medizintechnischen Einsatzart zugeordneten Einsatzorts des mobilen medizintechnischen Geräts. Das Verfahren 100 umfasst ferner einen Schritt S106 des Trainierens eines dynamischen Kollisionsmodells für das mobile medizintechnische Gerät. Das Trainieren S106 erfolgt mittels maschinellen Lernens (ML) und basierend auf der Kombination des erfassten S102 Hinweises auf die Einsatzart und die erfasste S104 Umgebungskarte mit dem Einsatzort.

Das Trainieren S106 (mittels ML) des dynamischen Kollisionsmodells wird in einer Planungsphase und/oder Installationsphase des mobilen medizintechnischen Geräts ausgeführt.

Optional umfasst das Verfahren 100 einen Schritt S101 des Vortrainierens S101 des dynamische Kollisionsmodells. Das Vortrainieren S101 kann insbesondere statisch sein und/oder anhand mindestens einer Umgebungskarte erfolgen.

Das Verfahren 100 kann ferner einen Schritt S105 des Erfassens von Positionsdaten hinsichtlich eines mobilen Hindernisses im Bereich der Umgebungskarte und/oder des Erfassens (z.B. von Positionsdaten hinsichtlich) des Einsatzorts des mobilen medizintechnischen Geräts umfassen.

Das Verfahren 100 kann ferner einen Schritt S108 des Erfassens von Umgebungssensordaten hinsichtlich des Einsatzortes während Testbewegungen des mobilen medizintechnischen Geräts umfassen.

Das Verfahren 100 kann ferner einen Schritt S110 des Validierens des trainierten S106 dynamischen Kollisionsmodells umfassen. Das Validieren S110 kann ein Abgleichen der erfassten S108 Umgebungssensordaten mit einem Zustand gemäß dem trainierten S106 dynamischen Kollisionsmodell umfassen.

Das Verfahren 100 kann ferner einen Schritt S111 des Nachtrainierens des dynamischen Kollisionsmodells umfassen. Das Nachtrainieren S111 kann insbesondere in einer Einsatzphase des mobilen medizintechnischen Geräts und/oder basierend auf realen historischen Bewegungsdaten erfolgen. Die (z.B. realen historischen) Bewegungsdaten können Kollisionsdaten umfassen.

Fig. 2 zeigt schematisch ein beispielhaftes Ablaufdiagramm eines (insbesondere computer-implementierten) Verfahrens 200 zum Steuern einer Bewegung eines mobilen medizintechnischen Geräts anhand eines dynamischen Kollisionsmodells.

Das Verfahren 200 umfasst einen Schritt S202 des Empfangens S202 eines Hinweises auf eine medizinische Einsatzart und einen zugeordneten Einsatzort eines mobilen medizintechnischen Geräts. Der Hinweis auf den zugeordneten Einsatzort kann implizit sein. Beispielsweise kann sich aus der medizinischen Einsatzart eindeutig der zugeordnete Einsatzort ergeben (z.B. für eine mobile Patientenliege, die einem fest installierten MRT zugeordnet ist).

Das Verfahren 200 umfasst ferner einen Schritt S204 des Erfassens von Umgebungssensordaten des mobilen medizintechnischen Geräts. Das Verfahren 200 umfasst ferner einen Schritt S206 des Auswertens der erfassten S204 Umgebungssensordaten und eine Schritt S208 des Bestimmens eines Steuersignals zum kollisionsfreien Steuern des mobilen medizintechnischen Geräts mittels eines (z.B. gemäß dem Verfahren 100) trainierten dynamischen Kollisionsmodells. Das Verfahren 200 umfasst ferner einen Schritt S210 des Bereitstellens des bestimmten S208 Steuersignals an eine Bewegungsaktorik des mobilen medizintechnischen Geräts.

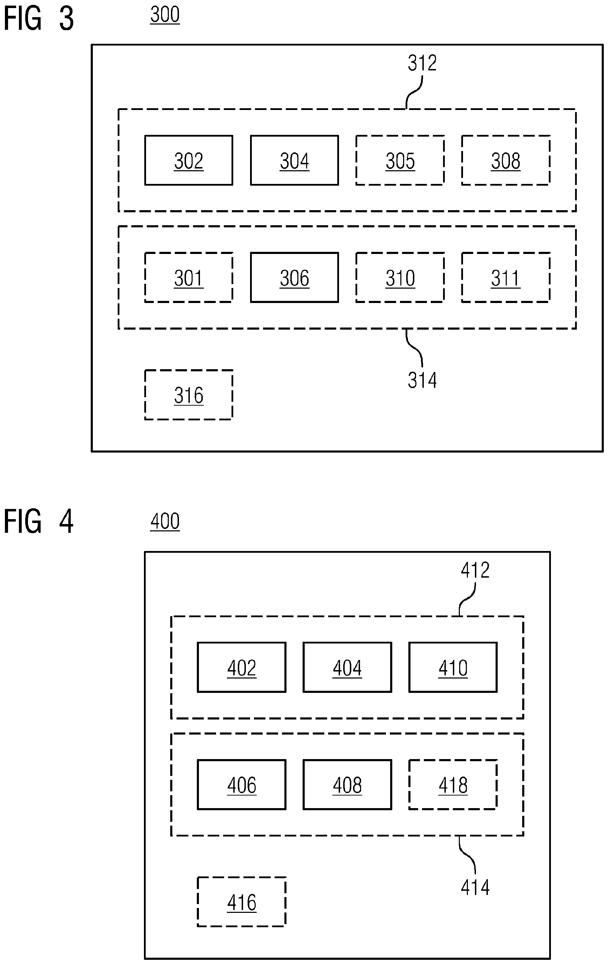

Fig. 3 zeigt schematisch eine Ausführungsbeispiel einer Trainings-Vorrichtung 300 zum Trainieren eines dynamischen Kollisionsmodells für ein mobiles medizintechnisches Gerät.

Die Trainings-Vorrichtung 300 umfasst eine Hinweis-Schnittstelle 302, die zum Erfassen eines Hinweises auf eine medizintechnische Einsatzart eines mobilen medizintechnischen Geräts ausgebildet ist. Die Trainings-Vorrichtung 300 umfasst ferner eine Umgebungskarten-Schnittstelle 304, die zum Erfassen einer Umgebungskarte eines der medizintechnischen Einsatzart zugeordneten Einsatzorts des mobilen medizintechnischen Geräts ausgebildet ist. Die Trainings-Vorrichtung 300 umfasst ferner eine Trainingseinheit 306, die zum Trainieren, mittels ML, eines dynamischen Kollisionsmodells für das mobile medizintechnische Gerät basierend auf der Kombination des erfassten Hinweises auf die Einsatzart und die erfasste Umgebungskarte mit dem Einsatzort ausgebildet ist. Das Trainieren (mittels ML) des dynamischen Kollisionsmodells ist in einer Planungsphase und/oder Installationsphase des mobilen medizintechnischen Geräts ausführbar.

Optional umfasst die Trainings-Vorrichtung 300 eine Vortrainingseinheit 301, die zum Vortrainieren des dynamische Kollisionsmodell, insbesondere statisch und/oder anhand mindestens einer Umgebungskarte, ausgebildet ist.

Die Trainings-Vorrichtung 300 kann ferner eine Hindernis-Schnittstelle 305 umfassen, die zum Erfassen von Positionsdaten hinsichtlich eines mobilen Hindernisses im Bereich der Umgebungskarte und/oder zum Erfassen (z.B. von Positionsdaten hinsichtlich) des Einsatzorts des mobilen medizintechnischen Geräts ausgebildet ist.

Die Trainings-Vorrichtung 300 kann ferner eine Umgebungssensordaten-Schnittstelle 308 umfassen, die zum Erfassen von Umgebungssensordaten hinsichtlich des Einsatzortes während Testbewegungen des mobilen medizintechnischen Geräts ausgebildet ist.

Die Trainings-Vorrichtung 300 kann ferner eine Validierungs-Einheit 310 umfassen, die zum Validieren des trainierten dynamischen Kollisionsmodells ausgebildet ist. Das Validieren kann ein Abgleichen der erfassten Umgebungssensordaten mit einem Zustand gemäß dem trainierten dynamischen Kollisionsmodell umfassen.

Die Trainings-Vorrichtung 300 kann ferner eine Nachtrainingseinheit 311 umfassen, die zum Nachtrainieren des dynamischen Kollisionsmodells ausgebildet ist. Das Nachtrainieren kann insbesondere in einer Einsatzphase des mobilen medizintechnischen Geräts und/oder basierend auf realen historischen Bewegungsdaten erfolgen. Die (insbesondere realen historischen) Bewegungsdaten können Kollisionsdaten umfassen.

Die Trainings-Vorrichtung 300 kann eine Eingabe-Ausgabe-Schnittstelle 312 umfassen. Die Hinweis-Schnittstelle 302, die Umgebungskarten-Schnittstelle 304, die optionale Hindernis-Schnittstelle 305 und/oder die optionale Umgebungssensordaten-Schnittstelle 308 können durch die Eingabe-Ausgabe-Schnittstelle 312 verkörpert bzw. in der Eingabe-Ausgabe-Schnittstelle 312 zusammengefasst sein.

Die Trainings-Vorrichtung 300 kann einen Prozessor 314 umfassen. Die Trainingseinheit 306, die optionale Vortrainingseinheit 301, die optionale Validierungs-Einheit 310 und/oder die optionale Nachtrainingseinheit 311 können durch den Prozessor 314 verkörpert bzw. in dem Prozessor 314 zusammengefasst sein.

Die Trainings-Vorrichtung 300 kann einen Speicher 316 umfassen. Beispielsweise können in dem Speicher 316 Programmelemente gespeichert sein, um das Verfahren 100 auszuführen. Alternativ oder ergänzend können in dem Speicher 316 Ergebnisse oder Zwischenergebnisse der Schritte des Verfahrens 100 (z.B. zwischen-) gespeichert werden.

Die Trainings-Vorrichtung 300 kann zum Ausführen des Verfahrens 100 ausgebildet sein.

Fig. 4 zeigt schematisch eine Ausführungsbeispiel eines Controllers 400 zum Steuern einer Bewegung eines mobilen medizintechnischen Geräts anhand eines dynamischen Kollisionsmodells.

Der Controller 400 umfasst eine Hinweis-Schnittstelle 402, die zum Empfangen eines Hinweises auf eine medizinische Einsatzart und einen zugeordneten Einsatzort eines mobilen medizintechnischen Geräts ausgebildet ist. Der Controller 400 umfasst ferner eine Umgebungssensordaten-Schnittstelle 404, die zum Erfassen von Umgebungssensordaten des mobilen medizintechnischen Geräts ausgebildet ist. Der Controller 400 umfasst ferner eine Auswerteeinheit 406, die zum Auswerten der erfassten Umgebungssensordaten ausgebildet ist. Der Controller 400 umfasst ferner eine Bestimmungseinheit 408, die zum Bestimmen eines Steuersignals zum kollisionsfreien Steuern des mobilen medizintechnischen Geräts ausgebildet ist. Das Bestimmen erfolgt mittels eines (z.B. mittels des Verfahrens 100 und/oder der Vortrainingseinheit 300) trainierten dynamischen Kollisionsmodells. Der Controller 400 umfasst ferner eine Steuersignal-Schnittstelle 410, die zum Bereitstellen des bestimmten Steuersignals an eine Bewegungsaktorik des mobilen medizintechnischen Geräts ausgebildet ist.

Der Controller 400 kann eine Validierungs-Einheit 418 umfassen, die zum Validieren des trainierten dynamischen Kollisionsmodells in der Installationsphase ausgebildet ist. Das Validieren kann ein Abgleichen erfasster Umgebungssensordaten (insbesondere hinsichtlich eines Einsatzortes) mit einem Zustand gemäß dem trainierten dynamischen Kollisionsmodell umfassen. Die erfassten Umgebungssensordaten für das Validieren können während Testbewegungen des mobilen medizintechnischen Geräts durch die

Umgebungssensordaten-Schnittstelle 404 erfasst werden.

Der Controller 400 kann eine Eingabe-Ausgabe-Schnittstelle 412 umfassen. Die Hinweis-Schnittstelle 402, die Umgebungssensordaten-Schnittstelle 404 und/oder die Steuersignal-Schnittstelle 410 können durch die Eingabe-Ausgabe-Schnittstelle 412 verkörpert und/oder in der Eingabe-Ausgabe-Schnittstelle 412 zusammengefasst sein.

Der Controller 400 kann einen Prozessor 414 umfassen. Die Auswerteeinheit 406, die Bestimmungseinheit 408 und/oder die optionale Validierungs-Einheit 418 können durch den Prozessor 414 verkörpert und/oder in dem Prozessor 414 zusammengefasst sein.

Der Controller 400 kann einen Speicher 416 umfassen. In dem Speicher 416 können beispielsweise Programmelemente gespeichert sein, um das Verfahren 200 und/oder das Validieren gemäß dem Verfahren 100 auszuführen. Alternativ oder ergänzend können in dem Speicher 416 316 Ergebnisse oder Zwischenergebnisse der Schritte des Verfahrens 200 und/oder des Validierens (z.B. zwischen-) gespeichert werden.

Der Controller 400 kann zum Ausführen des Verfahren 200 ausgebildet sein.

Ein mobiles medizintechnisches Gerät kann einen Controller 400 umfassen.

Die erfindungsgemäße Technik (z.B. umfassend das Verfahren 100, das Verfahren 200, die Trainings-Vorrichtung 300, den Controller 400 und/oder das mobile medizintechnische Gerät) kann auch als ML-basierte Kollisionsvorbeugung und -vermeidung hinsichtlich medizinischer Geräte bezeichnet werden.

Das erfindungsgemäß trainierte Kollisionsmodell wird nicht manuell statisch während der Entwicklung erzeugt, sondern automatisch mit Hilfe von ML-Verfahren bei der Planungs- und Installationsphase des Gerätes erlernt. Hierdurch kann das Kollisionsverhalten dynamisch an die spezifische Einsatzart und den zugeordneten Einsatzort (und/oder an eine Raum- und Aufnahmesituation) angepasst werden.

Die Implementierung von Kollisionsmodellen (auch: Modellen für einen Kollisionsrechner) in medizintechnischen Geräten mit automatischen Bewegungsfunktionen bringt herkömmlicherweise eine Vielzahl von kritischen Herausforderungen mit sich. Die Sicherheit des Patienten muss gewährleistet werden, indem verhindert wird, dass das Gerät in Kontakt beispielsweise mit einem Patienten oder anderen kritischen Komponenten kommt, was zu Verletzungen oder Beschädigungen führen könnte. Durch die Verwendung eines vereinfachten Modells, das beispielsweise auf einer Hüllkurve aus Kugeln basiert, wird herkömmlicherweise sichergestellt, dass die Kollisionsberechnungen innerhalb der engen zeitlichen Vorgaben der Steuerungszykluszeit durchgeführt werden können. Dies ist entscheidend, da Verzögerungen in der Reaktionszeit zu unerwünschten Kollisionen führen könnten.

Die Optimierung und Validierung des Kollisionsmodells ermöglichen eine effizientere und präzisere Positionierung des medizintechnischen Geräts, was zu einem erweiterten Bewegungsraum (auch: Feature-Space) führt und zu einer verbesserten Performance für die Durchführung des Bestimmens von Steuersignalen (und/oder von Berechnungsanfragen).

Durch eine detaillierte händische Optimierung eine rechnerunterstützten Konstruktions- (fachsprachlich: Computer-Aided-Design, CAD) Modells für die integrierte (auch: embedded) Hardware wird herkömmlicherweise sichergestellt, dass eine motorisch unterstütze Bewegungssteuerung eines medizintechnischen Geräts (auch: System) auch unter beschränkten Ressourcenbedingungen einer eingebetteten Steuerung effizient arbeitet. Dies ist ein aufwändiger manueller Arbeitsschritt, welcher herkömmlicherweise entscheidend ist, um die Integrität des Systems zu gewährleisten und gleichzeitig die notwendige Performance für Echtzeitoperationen zu liefern.

Durch die Echtzeitanforderung wird deutlich, dass eine schnelle und effiziente Bestimmung von Steuersignalen (auch: Berechnung) von zentraler Bedeutung ist, da das Kollisionsmodell für Echtzeit-Kollisionsprüfungen eingesetzt wird. Das bedeutet, dass sämtliche relevanten Kollisionskombinationen innerhalb eines sehr kurzen Zeitraums analysiert werden müssen. Bei einer Steuerungszykluszeit von rund 50 ms steht beispielsweise lediglich ein Zeitfenster von 10 ms für die Kollisionsberechnungen zur Verfügung, welches die maximale Dauer für alle notwendigen Berechnungen darstellt. Durch den herkömmlichen manuellen Anpassungsprozess des Entwicklers bestehen Gefahren hinsichtlich einer fehlenden Abdeckung von seltenen Spezialfällen, einer Übermodellierung sowie eines Zeitaufwands und einer Konsistenz.

Der Mensch als Gewohnheitstier neigt dazu, sich auf häufig auftretende Szenarien zu konzentrieren, dabei können seltene und unerwartete Kollisionsfälle (auch: Spezialfälle) bei der Modellierung übersehen werden. Dies wird im Allgemeinen herkömmlicherweise mittels eines Systemtests im Anschluss verifiziert. Eine mangelnde Abdeckung von seltenen Spezialfällen kann zu unerwünschten Kollisionen in realen Anwendungen führen, besonders in komplexen Umgebungen oder unter unvorhergesehen Bedingungen.

Eine Übermodellierung bzw. eine zu detaillierte Gestaltung des Kollisionsmodells führt herkömmlicherweise zu unnötigen Rechenaufwand. Die manuelle Modellierung ist ferner zeitaufwendig, da die Optimierung oft über mehrere Iterationen hinweg durchgeführt werden muss. Außerdem kann je nach Entwickler das Ergebnis in Aufwand und Qualität bzw. in einer Konsistenz variieren.

Herkömmlicherweise erfolgen die manuelle Erstellung, Optimierung und Tests der Kollisionsmodelle durch die Entwickler. Die Entwickler erstellen ein Kollisionsmodell herkömmlicherweise basierend auf ihrer Erfahrung und ihrem Fachwissen und anpassen es entsprechend an.

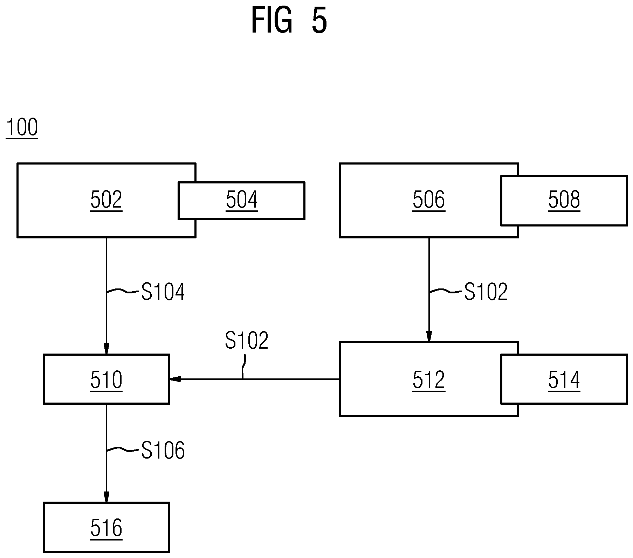

Ein Ausführungsbeispiel des Verfahrens 100 zum Trainieren des dynamischen Kollisionsmodells (auch: zur Modellerzeugung und/oder zur Erzeugung eines ML-Modells) ist in Fig. 5 schematisch gezeigt.

Es ist zu berücksichtigen, dass das erfindungsgemäße Trainings-Verfahren 100 vor der Anwendung durch den Benutzer stattfindet und ein dynamisches Kollisionsmodell erzeugt, welches zur Laufzeit (auch: in einer Inferenzphase) für die Kollisionsvermeidung herangezogen wird.

Wie in Fig. 5 schematisch skizziert, empfängt eine Gerätesteuerung 512 mit Sensorik 514 in der Planungsphase und/oder Installationsphase (auch: in einem Lernprozess) Daten bezüglich einer Einsatzart (z.B. einer Aufnahmesituation) 506 und positioniert das medizintechnische Gerät dementsprechend (z.B. virtuell oder reell). Die Daten bzgl. der Einsatzart (bzw. Aufnahmesituation) 506 können aus einer Datenbank 508 mit medizinischen Einsatzarten (bzw. Applikationen) entnommen werden. Bei der Installation des medizintechnischen Geräts kann die Datenbank 508 mit verschiedenen medizinischen Einsatzarten (auch: Applikationen) verfügbar sein. Die Datenbank 508 kann beispielsweise Informationen über die spezifischen Anforderungen und Einschränkungen jeder Einsatzart (auch: Applikation) enthalten.

Mithilfe der zur Verfügung stehenden Sensorik 514 können zum Trainingszeitpunkt Rückschlüsse auf Position und Umgebung des medizintechnischen Geräts getroffen und für den weiteren Lernprozess bzw. das weitere Trainieren herangezogen werden. Das Trainieren kann am realen System oder komplett virtuell durchgeführt werden.

Bei der Planung des medizintechnischen Geräts wird eine Datenbank 504 mit einem oder mehreren verschiedenen Raumplänen erstellt. Die Raumpläne enthalten beispielsweise Informationen über die Raumgeometrie, Hindernisse und andere relevante Merkmale. Die Datenbank 504 kann von einem virtuellen Raumplaner 502 erstellt werden. Alternativ oder ergänzend kann der virtuelle Raumplaner 502 dazu verwendet werden, für eine erfasste (bzw. ausgewählte) medizinische Einsatzart 506 einen Raumplan mit zugeordnetem Einsatzort auszuwählen (bzw. bereitzustellen).

Der an Bezugszeichen 510 ML-Algorithmus im Ausführungsbeispiel der Fig. 5 lernt aus den Daten der Raumpläne 504 und den Daten der medizinischen Applikationen 508, um ein dynamisches Kollisionsmodell 516 zu erstellen bzw. zu trainieren. Mit Hilfe des erzeugten bzw. trainierten dynamischen Kollisionsmodells 516 können zur Laufzeit bzw. in der Inferenzphase Entscheidungen hinsichtlich einer Bewegungssteuerung des mobilen medizintechnischen Geräts getroffen werden.

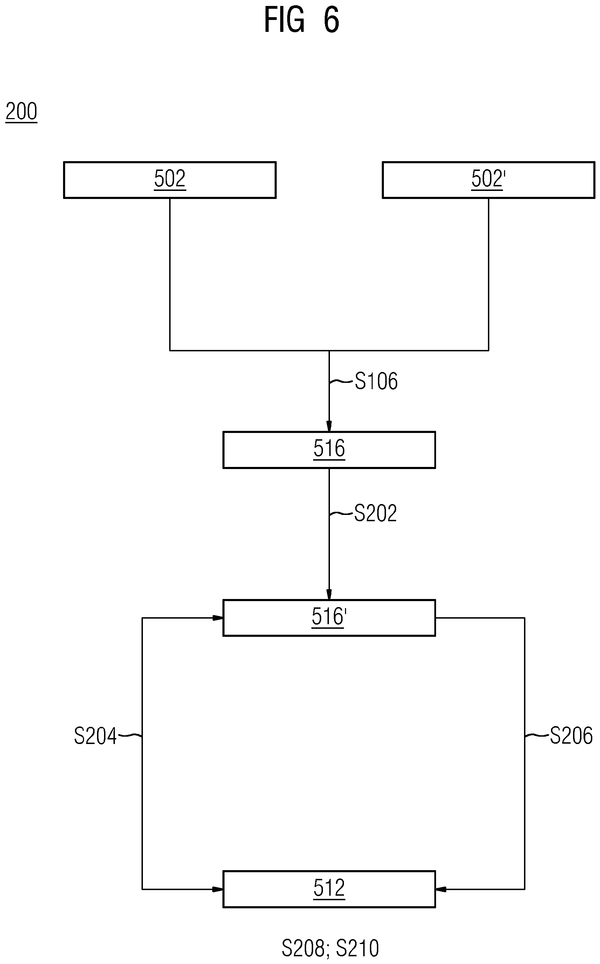

Ein Ausführungsbeispiel des Verfahrens 200 zum Steuern einer Bewegung eines mobilen medizintechnischen Geräts anhand eines dynamischen Kollisionsmodells zur Laufzeit bzw. in der Inferenzphase ist in Fig. 6 schematisch gezeigt. Das dynamische Kollisionsmodell 516 kann mittels des Verfahrens 100 gemäß Fig. 5 oder Fig. 1 trainiert sein.

Die Gerätesteuerung 512 greift für die Kollisionsvermeidung auf das generierte bzw. trainierte Kollisionsmodell 516` zu. Die Gerätesteuerung 512 kann unter zu Hilfenahme des Kollisionsmodells 516' Positionierungsentscheidungen (beispielsweise unter Verwendung von erfassten S204 Umgebungsdaten) treffen. Als Input für das Kollisionsmodell 516` dienen die Positionierungsanfrage und die Einsatzart (bzw. gewünschte Aufnahmesituation) 506. Der für die Einsatzart 506 verwendete Teil des allgemeinen trainierten dynamischen Kollisionsmodells 516 kann beispielsweise das Kollisionsmodell 516' sein, auf das die Gerätesteuerung 512 zugreift.

Das Kollisionsmodell 516` wertet S206 die empfangenen (bzw. erfassten) Daten (insbesondere der empfangenen S202 Einsatzart 506, eines Umgebungsplans 502' des zugeordneten Einsatzorts und/oder der erfassten S204 Umgebungsdaten) aus.

Unter Berücksichtigung der empfangenen S202; S204 und ausgewerteten S206 Daten bestimmt (bzw. berechnet) S208 die Gerätesteuerung 512 den optimalen Pfad für die Positionierung des Geräts, wobei sie sicherstellt, dass das Gerät sicher und effizient zu seinem Ziel bewegt wird, ohne Kollisionen zu riskieren. Auch bei benutzergestützter Bewegungsteuerung kann das Kollisionsmodell 516` Kollisionen verhindern, indem es die erlaubte Geschwindigkeit in jedem Raumpunkt erlernt hat und der Gerätesteuerung 512 zurückliefert. Dieses Ausführungsbeispiel ermöglicht es dem Gerät, sich dynamisch an die gerätespezifischen Gegebenheiten sowie situationsbedingten Anforderungen der Einsatzart (z.B. Aufnahmesituation) 506 anzupassen.

Ein Hauptunterschied zwischen der erfindungsgemäßen Technik und herkömmlichen Lösungen liegt in der automatischen Anpassungsfähigkeit und Lernfähigkeit des dynamischen Kollisionsmodells bzw. Systems durch den Einsatz von ML. Während konventionelle Ansätze auf festen vordefinierten Parametern basieren und manuelle Eingriffe erfordern, ermöglicht die ML-basierte Lösung eine dynamische und kontinuierliche Anpassung an die Umgebung unter Berücksichtigung der Raumplanung, sowie der gegebenen Aufnahmesituation.

Statt sich auf vordefinierte Modelle zu verlassen, nutzt das ML-System Daten aus der realen Umgebung, um ein Kollisionsmodell zu erstellen und bezieht dazu noch die aktuelle medizinische Anwendung mit ein. Dies ermöglicht eine präzise (insbesondere datengetriebene) Anpassung an die tatsächlichen Gegebenheiten.

Das System lernt während der Installationsphase und/oder Planungsphase bzw. das dynamische Kollisionsmodell wird während der Installationsphase und/oder Planungsphase trainiert. Dies kann auch als vordefiniertes Lernen bezeichnet werden. Das System bzw. das dynamische Kollisionsmodell passt sich an die vorliegenden Gegebenheiten an und lernt dadurch, sich in entsprechend dieser Situation effizienter und/oder auch zuverlässiger zu verhalten.

Bei Erkennung eines Fehlers oder einer Kollision können die Erfahrungswerte für die Anpassung zukünftiger Modelle gesammelt werden. So kann das System bzw. das dynamische Kollisionsmodell über die Generationen hinweg aus den Erfahrungswerten weiterwachsen und sich verbessern. Dies kann auch als Selbstkorrektur des Systems bzw. des dynamischen Kollisionsmodells bezeichnet werden.

Während konventionelle Systeme manuelle Eingriffe durch den Benutzer erfordern, ermöglicht das ML-System bzw. das mittels ML trainierte dynamische Kollisionsmodell eine automatisierte Anpassung, wodurch der Bedarf an menschlichen Eingriffen minimiert wird. Dadurch wird eine verbesserte Automatisierung der Bewegung (auch: Fahrt) des mobilen medizinischen Geräts verbessert.

Das ML-System bzw. das mittels ML trainierte dynamische Kollisionsmodell kann mit verschiedenen Algorithmen und Datenquellen arbeiten, was es flexibel, vielseitig und anpassungsfähig an unterschiedliche Gerätetypen und Anwendungen macht. So kann beispielsweise Applikationswissen (bzw. Wissen über medizintechnische Einsatzarten) Geräte-übergreifend verwendet werden.

Der Vorteil der erfindungsgemäßen Technik basiert insbesondere auf der Fähigkeit des ML, aus Daten (z.B. hinsichtlich Einsatzart, Einsatzort und/oder Umgebung) zu lernen und sich an veränderliche Umgebungen im medizinischen automatisierten Positionierungskontext anzupassen, was eine deutliche Verbesserung gegenüber den starren manuell konfigurierten herkömmlichen Systemen bzw. Kollisionsmodellen darstellt.

Charakteristisch für die erfindungsgemäße Technik ist, dass sich das trainierte Kollisionsmodell und daraus resultierende Kollisionsverhalten des mobilen medizintechnischen Geräts dynamisch an Raumsituation (bzw. Umgebung) und die Einsatzart (z.B. Aufnahmesituation) anpasst.

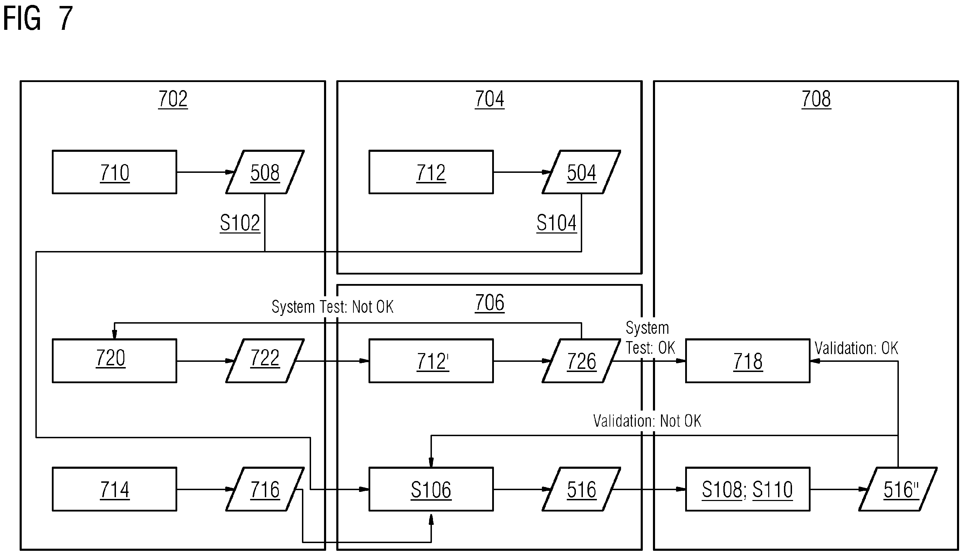

Fig. 7 zeigt ein erweitertes Flussdiagramm zum erfindungsgemäßen Erstellen und Einsetzen eines dynamischen Kollisionsmodells für ein mobiles medizintechnisches Gerät. Das Flussdiagramm in Fig. 7 zeigt eine (z.B. allgemeine) Entwicklungsphase 702, eine (z.B. kundenspezifische) Planungsphase 704, eine Test- und/oder Trainingsphase 706 und eine Installationsphase 708 und/oder Inferenzphase 708.

In der (z.B. allgemeinen) Entwicklungsphase 702 wird eine Datenbank 508 für medizintechnische Einsatzarten (z.B. umfassend medizinische bildgebende Geräte und/oder zugehörige klinische Protokolle) konstruiert 710. Ebenfalls in der (z.B. allgemeinen) Entwicklungsphase 702 findet eine Softwareentwicklung 720 statt, die als Ergebnis eine Systemsteuerungs-Software 722 hat. Parallel wird während der (z.B. allgemeinen) Entwicklungsphase 702 ein (z.B. statisches und/oder vortrainiertes) Kollisionsmodell (auch: insbesondere untrainiertes oder nur vortrainiertes, ML-Modell) 716 erstellt 714. In der Test- und/oder Trainingsphase (auch: Lernphase) 706 erfolgt ein (insbesondere kundenspezifisches) Trainieren S106, um das trainierte dynamische Kollisionsmodell 516 zu erhalten. Außerdem werden in der Test- und/oder Trainingsphase 706 Tests und/oder Simulationen für die Systemsteuerungs-Software 722 vorgenommen 712` und Funktionstestergebnisse 726 erzielt.

In der der Test- und/oder Trainingsphase 706 vorhergehenden (z.B. kundenspezifische) Planungsphase 704 wird eine Systemsteuerungs-Software für korrekte Arbeitsfunktionalitäten getestet und/oder simuliert 712 und eine Datenbank 504 für einen (z.B. kundenspezifischen) Raumplan erstellt.

Der Begriff Systemsteuerungs-Software kann eine Steuerung und/oder Regelung (kollektiv auf Englisch: control) des Systems mittels Software umfassen.

Sowohl die Datenbank 508 für medizintechnische Einsatzarten aus der (z.B. allgemeinen) Entwicklungsphase 702 als die Datenbank 504 für einen (z.B. kundenspezifischen) Raumplan geht ins Trainieren S106 in der Test- und/oder Trainingsphase 706, um das trainierte dynamische Kollisionsmodell 516 zu erhalten.

In der Installationsphase (und/oder Inferenzphase) 708 wird das dynamische Kollisionsmodell 516 (insbesondere anhand erfasster S108 Umgebungsdaten während Testbewegungen und/oder in der kundenspezifischen Umgebung) validiert S110. Hierdurch wird Sicherung und Validierung des Kollisionsmodells 516 anhand von Sensor-Feedback (z.B. umfassend Umgebungssensordaten) erreicht. Das validierte dynamische Kollisionsmodell 516" wird zusammen mit weiterer Software (beispielsweise Software zur Geräte- bzw. Bewegungssteuerung) eingesetzt 718, sofern das Funktionstestergebnis 726 befriedigend oder fehlerfrei ist.

Wenn das Funktionstestergebnis 726 unbefriedigend oder fehlerbehaftet ist, wird die Softwareentwicklung 720 fortgesetzt.

Die Test- und/oder Trainingsphase 706 zwischen der Erstellung des Raumplans für den Kunden (z.B. einen Betreiber einer medizinischen Einrichtung) und der Installation beim Kunden (z.B. in der medizinischen Einrichtung) kann bereits beim Kunden (und/oder in der medizinischen Einrichtung) stattfinden.

Anhand des Raumplans, welcher die Positionen und Orientierungen der einzelnen Systemkomponenten (z.B. umfassend das mobile medizintechnische Gerät und/oder weitere Geräte im Raum) beschreibt, lernt S106 das dynamische Kollisionsmodell die Kollisionsfälle und deren Vermeidungsstrategie.

Anhand der medizinischen Einsatzart (z.B. Aufnahmesituation) 508 wird der Anwendungsfall direkt mitberücksichtigt. Hierzu kommen bewegliche Objekte hinzu, welche für die Applikation benötigt werden (z.B. ein Patienten-Stand und/oder eine Armhalterung). Hinzu kommt beispielsweise die jeweilige Patientenposition, welche detaillierter als bei konventionellen Modellen berücksichtigt werden kann.

Das für den jeweiligen Kunden (bzw. die jeweilige medizinische Einrichtung) trainierte (bzw. generierte) dynamische Kollisionsmodell (auch: ML-Modell) 516 wird während der Installationsphase validiert, insbesondere durch zusätzliche Sensorik.

Das finale erstellte und/oder validierte Kollisionsmodell (auch: ML-Modell) 516" wird von der Systemsteuerungs-Software (fachsprachlich: System Control Software) 722 verwendet, um eine Kollisionsvermeidung durchzuführen. Hierfür wird eine Risikobetrachtung vorgenommen, um weitere Fehlersicherheit zu gewährleisten. Insbesondere wird eine Kombination aus Sensorik und dynamischen Kollisionsmodell (auch: ML-Modell) analysiert, um eine angemessene (z.B. eine Minimalschwelle überschreitende) Risikoakzeptanz zu erreichen.

Das Trainieren (auch: Erstellen) des (z.B. statischen und/oder dynamischen) Kollisionsmodells kann mit Hilfe eines verstärkenden Lern- (fachsprachlich: Reinforcement Learning) Algorithmus unterstützt bzw. das Kollisionsmodell getestet und optimiert werden.

Das Kollisionsmodell kann vom Entwickler entworfen werden. Der RL-Algorithmus (und/oder das RL-Modell) hat die Aufgaben, die Vollständigkeit des Kollisionsmodells sicherzustellen, indem es nahezu alle Kombinationen von möglichen Kollisionen überprüft und adaptiert; die optimale Rechenzeit zu erreichen, welche eine erlaubte Obergrenze an Rechenzeit nicht überschreitet; und zwar bei einem gleichzeitig optimalen Bewegungsbereich für das Gerät.

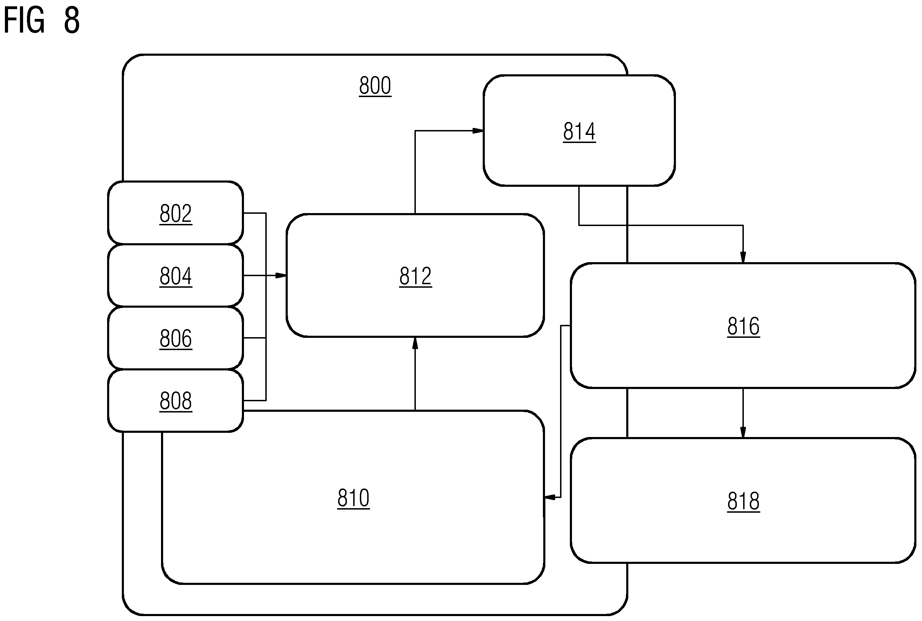

Der beispielhaft in Fig. 8 gezeigt RL-Algorithmus verwendet einen Agenten (auch: Robot Agent) 800 zur Kollisionserkennung. Zum Trainieren des RL-Algorithmus wird ein System 810 aus Zuständen (fachsprachlich: States) und Belohnungen (fachsprachlich: Rewards) verwendet. Ein Zustand des mobilen medizintechnischen Geräts (und optional seiner Umgebung, fachsprachlich: Environment) umfasst eine aktuelle Positionierung und Orientierung des mobilen medizintechnischen Geräts (kurz auch: Roboters) und optional weiterer Geräte, eine Konfiguration des Kollisionsmodells oder ein aktueller Kollisionszustand, Modellabschnitte mit hoher Rechenleistung, unnötige Zwischenräume, wie etwas tote Winkel, und/oder Informationen über die Umgebung, welche den Bewegungsraum des medizinischen Roboters abdeckt. Die Belohnungen werden dafür angewendet, um das Lernverhalten in die gewünschte Richtung zu lenken und so durch positive und negative Bestärkung bestimmter Aktionen einen "Lerneffekt" zu erzielen. Die Belohnungen zielen insbesondere auf das Entdecken von bisher nicht abgedeckten Kollisionsfällen und Modellabschnitten mit hohem Rechenaufwand ab.

Die Umgebung (auch: Environment) 816 kann einen Zustand des kompletten Robotersystems umfassen sowie einen Kollisionsstatus. Das komplette Robotersystem kann ein Roboter-Modell, ein Welt-Modell und ein Kollisionsmodell umfassen.

An Bezugszeichen 812 in Fig. 8 ist eine Strategie (fachsprachlich: Policy) gezeigt, die basierend auf dem aktuellen Zustand (bzw. dem System 810 mit dem Zustand) Entscheidungen der auszuführenden Bewegungen (fachsprachlich auch: Action) des Geräts (bzw. Roboters) trifft.

Die Strategie kann eine Menge Regeln umfassen, die durch eine Kombinatorik aus den Rückgabewerten von Umgebung, den erhaltenen Belohnungen und dem eigenen internen Zustand abgeleitet werden.

Die Strategie 812 bildet den zentralen Kern des lernenden Systems bzw. Agenten 800 in dem Ausführungsbeispiel der Fig. 8. Mit Hilfe der Strategie (auch: Entscheidungsrichtlinien bzw. -regeln) kann der Agent 800 (bzw. das mobile medizintechnische Gerät oder Robotersystems, kurz: Systems) eigenständig wählen, was der aus der aktuellen Sicht nächste sinnvolle Schritt (und/oder die nächste sinnvolle Bewegung) ist. Durch die Rückmeldung 810 über die Umgebung, den Zustand und die Belohnung können Strategien 812 verändert werden, um so anhand der getroffenen Entscheidung zu lernen, ob diese den gewünschten Erfolg erzielt hat.

Aktionen (auch: Actions) 814 sind Bewegungsprimitive aller Achsen in allen möglichen Richtungen, welche motorische Bewegungen auslösen und so das Robotersystem anhand der Freiheitsgrade durch den Raum steuern können. Die Aktionen 814 werden anhand der zuvor erlernten Strategie 812 gewählt und bilden so das Bindeglied zwischen der lernenden und der ausführenden Komponente ab.

An Bezugszeichen 818 in Fig. 8 ist eine Terminierung gezeigt. Es werden keine weiteren unentdeckten oder unabgedeckten Kollisionsfälle gefunden. Keine weiteren signifikanten Optimierungen des Kollisionsmodells sind möglich und/oder das Kollisionsmodell konvergiert.

Zusätzlich zu den oben beschriebenen Komponenten kann der Agent 800 (bzw. das Modell) mit Hilfe von Hyperparametertuning angepasst werden, um beispielsweise einen schnelleren Lernerfolg zu erzielen. In Fig. 8 sind exemplarisch dargestellt "Value Function" 802, "Discount Factor" 804, "Learning Rate" 805 und "Exploration x Exploitation" 808 für das Hyperparametertuning.

Ein wichtiger Unterschied zwischen der erfindungsgemäßen Technik gemäß dem Ausführungsbeispiel der Fig. 8 und herkömmlichen Lösungen liegt in der in der Anwendung von RL zur Erstellung, zum Test und zur Optimierung von (z.B. zumindest anfänglich statischen) Kollisionsmodellen. Während konventionelle Ansätze sich auf manuelle Modellierung und Optimierung durch menschliche Entwickler verlassen, nutzt die erfindungsgemäße Technik die automatisierte Lernfähigkeit von RL, um das Kollisionsmodell zu verbessern.

RL-Algorithmen sind darauf spezialisiert, unbekannte Umgebungen zu analysieren und dabei optimale Strategien zu definieren. Im Kontext des Kollisionsmodells bedeutet dies, dass der Algorithmus systematisch verschiedene Kollisionsszenarien testen und gleichzeitig die besten Modelleigenschaften lernen kann. Dies kann auch als automatisierte Exploration und Exploitation bezeichnet werden.

RL kann kontinuierlich Feedback aus einer realen und/oder simulierten Umgebung nutzen, um das Modell zu verbessern. Dies ermöglicht eine dynamische Anpassung und Optimierung (auch: adaptives Lernen) des Kollisionsmodells über die Zeit.

Durch die Definition von Belohnungen (bzw. Rewards) für bestimmte Kriterien, wie beispielsweise die Vermeidung von Kollisionen und die Einhaltung von Rechenzeitgrenzen, kann das RL-Modell spezifisch darauf trainiert werden, diese Ziele zu erreichen. Dies kann als belohnungsbasierte Optimierung bezeichnet werden.

Ein gut trainiertes RL-Modell kann seine gelernten Strategien auf neue, bisher unbekannte Szenarien anwenden, was eine robustere und flexiblere Lösung im Vergleich zu fest programmierten oder manuell erstellten Modellen bietet. Anders ausgedrückt ist das RL-trainierte Kollisionsmodell generalisierbar.

Ein Vorteil der erfinderischen Technik basiert auf der Fähigkeit des RL, automatisch und adaptiv optimale statische Kollisionsmodelle zu erstellen, die sowohl sicher als auch effizient in Bezug auf die Rechenzeit sind. Dies stellt einen signifikanten Fortschritt gegenüber konventionellen, manuell erstellten Modellen dar.

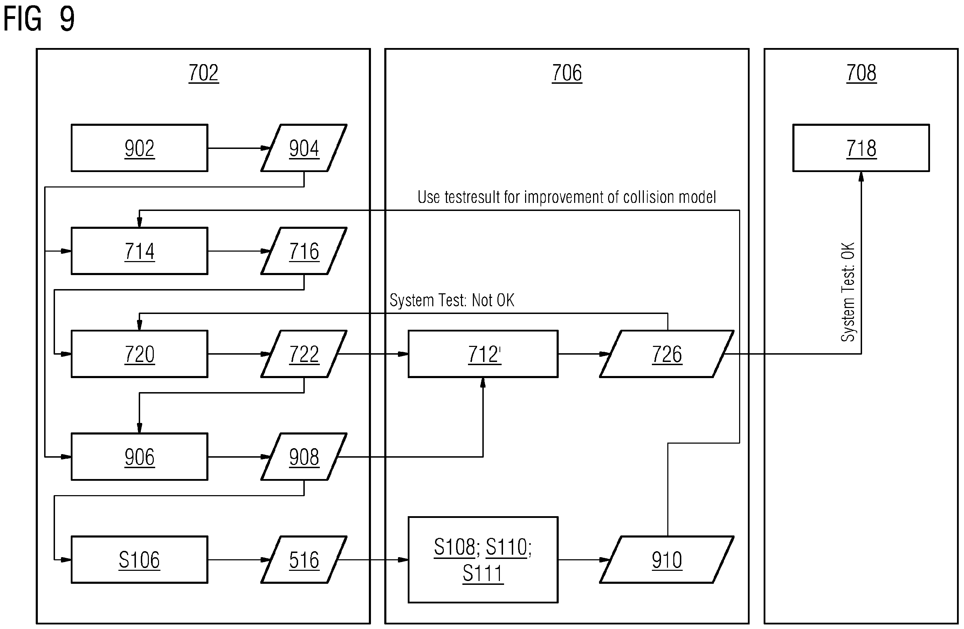

Fig. 9 zeigt ein Ausführungsbeispiel der erfindungsgemäßen Technik zur Kollisionsvermeidung bei Verwenden von RL. Die gleichen Schritte und Zwischenergebnisse wie in den vorstehenden Figuren, insbesondere Fig. 7, sind mit denselben Bezugszeichen bezeichnet.

An Bezugszeichen 902 in Fig. 9 werden in der Entwicklungsphase 702 mechatronische Roboterteile für ein medizintechnisches Gerät aus oder für ein CAD-Modell erzeugt. Das erzeugte Modell kann ein Bestandteil der Systemsteuerungs-Software (fachsprachlich: System Control Software) sein.

An Bezugszeichen 906 wird ein digitaler Zwilling 908 bereitgestellt. Der digitale Zwilling 908 im Ausführungsbeispiel der Fig. 9 verwendet das CAD-Modell 904 als visuelle Hülle und Systemsteuerungs-Software (fachsprachlich: System Control Software).

Das Kollisionsmodell (auch: RL-Modell) 516 wird während der Entwicklungsphase 702 erzeugt. Die Basis des Kollisionsmodelld (bzw. RL-Modells) 516 ist ein digitaler Zwilling (fachsprachlich: Digital Twin) 908, welcher an Bezugszeichen 906 erzeugt bzw. bereitgestellt wird und Informationen über das erstellte (z.B. statische und/oder höchstens vortrainierte) Kollisionsmodell 716 und über das CAD-Modell 904 zur Verfügung hat. Der digitale Zwilling 908 gibt entsprechendes Feedback, welche Art von Kollision auftritt, wenn zwei oder
mehr mechatronisch bewegliche Elemente zusammenstoßen.

Im Training S106, um das dynamische Kollisionsmodell 516 zu erhalten, können drei verschiedene Fälle vorkommen. Erstens kann aus dem Kollisionsmodell 716 ein "normaler" Kollisionsfall abgeleitet werden. Hierbei kann eine Rechenleistung mit gemessen werden für ein zukünftige Optimierung. Zweitens kann mittels der Kombination des Kollisionsmodells 716 mit dem CAD-Modell 904 ein teilweise abgedeckter Kollisionsfall abgeleitet werden. Der teilweise abgedeckte Kollisionsfall muss während der Entwicklungsphase 702 berücksichtigt werden, um eine Kollisionsmodellanpassung zu ermöglichen. Drittens kann aus dem CAD-Modell 904 ein komplett nicht abgedeckter Kollisionsfall abgeleitet werden. Der komplett nicht abgedeckte Kollisionsfall muss während der Entwicklungsphase 702 berücksichtigt werden, um eine Kollisionsmodellanpassung zu ermöglichen. Auch Teile des Kollisionsmodells, welche nie kollidiert haben können während einer Optimierung wieder mitberücksichtigt werden.

An Bezugszeichen 910 ist ein Kollisionsqualitäts- und Fallabdeckungstestergebnis eines Tests S108, einer Validierung S110 und eines Nachtrainings S111, bei dem das dynamische Kollisionsmodell (auch: RL-Modell) 516 fähig ist, schwache und nicht abgedeckte Bereiche im Kollisionsmodell 716 zu finden, schematisch dargestellt. Aufgrund des Kollisionsqualitäts- und Fallabdeckungstestergebnis 910 kann die Konstruktion 714 des Kollisionsmodells verbessert werden.

Anhand des Feedbacks vom digitalen Zwilling 908 lernt das Kollisionsmodell 516, wie es am besten Kollisionsfälle findet und dadurch auch aktiv nach schwach abgedeckten Kollisionsfällen sucht.

Der verwendete RL-Algorithmus ist für die Durchführung nicht entscheidend und muss nicht explizit mit betrachtet werden. Verschiedene RL-Algorithmen können die Problemstellung lösen.

Mit Hilfe der Ergebnisse aus dem Lernprozess und/oder Optimierungsprozess des Kollisionsmodells (auch: RL-Modells) 716; 516 kann das Kollisionsmodell 716 entsprechend optimiert werden.

In einer weiterführenden Ausbildung der erfinderischen Technik kann ein Multi-Agent-RL-Ansatz mit einem konkurrierenden Netzwerk den Optimierungsschritt bzw. Optimierungsprozess ausführen.

Unabhängig vom grammatikalischen Geschlecht eines bestimmten Begriffs sind Personen (z.B., Benutzer, Bediener, Patienten und/oder medizinisches Personal) mit männlicher, weiblicher oder anderer Geschlechteridentität mit umfasst.

Soweit nicht bereits explizit beschrieben, können einzelne Ausführungsformen oder deren einzelne Aspekte und Merkmale, die in Bezug auf die Zeichnungen beschrieben sind, miteinander kombiniert oder ausgetauscht werden, ohne den Umfang der beschriebenen Erfindung einzuschränken oder zu erweitern, wenn eine solche Kombination oder ein solcher Austausch sinnvoll und im Sinne der vorliegenden Erfindung ist. Vorteile, die in Bezug auf eine bestimmte Ausführungsform der vorliegenden Erfindung oder in Bezug auf eine bestimmte Figur beschrieben sind, sind, wo immer dies zutrifft, auch Vorteile anderer Ausführungsformen der vorliegenden Erfindung.

## Patentansprüche

1. Computer-implementiertes Verfahren (100) zum Trainieren eines dynamischen Kollisionsmodells für ein mobiles medizintechnisches Gerät, umfassend die Schritte:
- Erfassen (S102) eines Hinweises auf eine medizintechnische Einsatzart eines mobilen medizintechnischen Geräts;
- Erfassen (S104) einer Umgebungskarte eines der medizintechnischen Einsatzart zugeordneten Einsatzorts des mobilen medizintechnischen Geräts; und
- Trainieren (S106), mittels maschinellen Lernens, ML, eines dynamischen Kollisionsmodells für das mobile medizintechnische Gerät basierend auf der Kombination des erfassten (S102) Hinweises auf die Einsatzart und die erfasste (S104) Umgebungskarte mit dem Einsatzort,
wobei das Trainieren (S106), mittels ML, des dynamischen Kollisionsmodells in einer Planungsphase und/oder Installationsphase des mobilen medizintechnischen Geräts ausgeführt wird.

2. Verfahren (100) gemäß Anspruch 1, wobei die medizintechnische Einsatzart eine Aufnahme von medizinischen Bilddaten ist, und/oder
wobei das mobile medizintechnische Gerät ein mobiles bildgebendes Gerät oder eine Komponente eines mobilen bildgebenden Gerätes ist, optional
wobei die Komponente ein C-Bogen oder ein Patiententisch ist.

3. Verfahren (100) gemäß einem der vorhergehenden Ansprüche, ferner umfassend mindestens einen der Schritte:
- Vortrainieren (S101) des dynamische Kollisionsmodells, insbesondere statisch, anhand mindestens einer Umgebungskarte;
- Erfassen (S105) von Positionsdaten hinsichtlich eines mobilen Hindernisses im Bereich der Umgebungskarte und/oder des Einsatzorts des mobilen medizintechnischen Geräts; und/oder
- Nachtrainieren (S111) des dynamischen Kollisionsmodells, insbesondere in einer Einsatzphase des mobilen medizintechnischen Geräts, basierend auf realen historischen Bewegungsdaten, wobei die Bewegungsdaten Kollisionsdaten umfassen.

4. Verfahren (100) gemäß einem der vorhergehenden Ansprüche, in der Installationsphase ferner umfassend die Schritte:
- Erfassen (S108) von Umgebungssensordaten hinsichtlich des Einsatzortes während Testbewegungen des mobilen medizintechnischen Geräts; und
- Validieren (S110) des trainierten (S106) dynamischen Kollisionsmodells, wobei das Validieren (S110) ein Abgleichen der erfassten (S108) Umgebungssensordaten mit einem Zustand gemäß dem trainierten (S106) dynamischen Kollisionsmodell umfasst.

5. Verfahren (100) gemäß einem der vorhergehenden Ansprüche, wobei beim Trainieren (S106), mittels ML, des dynamischen Kollisionsmodells mindestens eine Randbedingung und/oder Zusatzbedingung berücksichtigt wird, optional
wobei die Randbedingung und/oder Zusatzbedingung eine vorbestimmte Bedingung an einen Bewegungsablauf umfasst, insbesondere eine Geschwindigkeitsvorgabe, und/oder
wobei die Randbedingung und/oder Zusatzbedingung eine Benutzerunterstützung einer Bewegung umfasst.

6. Verfahren (100) gemäß einem der vorhergehenden Ansprüche, wobei das ML ein verstärkendes Lernen, RL, und/oder das Trainieren (S106) des Kollisionsmodells einen digitalen Zwilling des mobilen medizintechnischen Geräts verwendet.

7. Verfahren (100) gemäß einem der vorhergehenden Ansprüche, wobei zumindest der Schritt des Trainierens (S106) des dynamischen Kollisionsmodells außerhalb des mobilen medizintechnischen Geräts ausgeführt wird.

8. Verfahren (100) gemäß einem der vorhergehenden Ansprüche, wobei das trainierte (S106) dynamische Kollisionsmodell lokal auf dem mobilen medizintechnischen Gerät gespeichert und ausgeführt wird.

9. Computer-implementiertes Verfahren (200) zum Steuern einer Bewegung eines mobilen medizintechnischen Geräts anhand eines dynamischen Kollisionsmodells, umfassend die Schritte:
- Empfangen (S202) eines Hinweises auf eine medizinische Einsatzart und einen zugeordneten Einsatzort eines mobilen medizintechnischen Geräts;
- Erfassen (S204) von Umgebungssensordaten des mobilen medizintechnischen Geräts;
- Auswerten (S206) der erfassten (S204) Umgebungssensordaten und Bestimmen (S208), mittels eines gemäß einem der Ansprüche 1 bis 8 trainierten dynamischen Kollisionsmodells, eines Steuersignals zum kollisionsfreien Steuern des mobilen medizintechnischen Geräts; und
- Bereitstellen (S210) des bestimmten (S208) Steuersignals an eine Bewegungsaktorik des mobilen medizintechnischen Geräts.

10. Trainings-Vorrichtung (300) zum Trainieren eines dynamischen Kollisionsmodells für ein mobiles medizintechnisches Gerät, umfassend:
- eine Hinweis-Schnittstelle (302), die zum Erfassen eines Hinweises auf eine medizintechnische Einsatzart eines mobilen medizintechnischen Geräts ausgebildet ist;
- eine Umgebungskarten-Schnittstelle (304), die zum Erfassen einer Umgebungskarte eines der medizintechnischen Einsatzart zugeordneten Einsatzorts des mobilen medizintechnischen Geräts ausgebildet ist; und
- eine Trainingseinheit (306), die zum Trainieren, mittels maschinellen Lernens, ML, eines dynamischen Kollisionsmodells für das mobile medizintechnische Gerät basierend auf der Kombination des erfassten Hinweises auf die Einsatzart und die erfasste Umgebungskarte mit dem Einsatzort ausgebildet ist,
wobei das Trainieren, mittels ML, des dynamischen Kollisionsmodells in einer Planungsphase und/oder Installationsphase des mobilen medizintechnischen Geräts ausführbar ist.

11. Trainings-Vorrichtung (300) gemäß dem direkt vorhergehenden Anspruch, ferner umfassend die Merkmale gemäß einem der Ansprüche 2 bis 8 und/oder ferner dazu ausgebildet, Verfahrensschritte gemäß einem der Ansprüche 2 bis 8 auszuführen.

12. Controller (400) zum Steuern einer Bewegung eines mobilen medizintechnischen Geräts anhand eines dynamischen Kollisionsmodells, umfassend:
- eine Hinweis-Schnittstelle (402), die zum Empfangen eines Hinweises auf eine medizinische Einsatzart und einen zugeordneten Einsatzort eines mobilen medizintechnischen Geräts ausgebildet ist;
- eine Umgebungssensordaten-Schnittstelle (404), die zum Erfassen von Umgebungssensordaten des mobilen medizintechnischen Geräts ausgebildet ist;
- eine Auswerteeinheit (406), die zum Auswerten der erfassten Umgebungssensordaten ausgebildet ist,
- eine Bestimmungseinheit (408), die zum Bestimmen, mittels eines gemäß einem der Ansprüche 1 bis 8 trainierten dynamischen Kollisionsmodells, eines Steuersignals zum kollisionsfreien Steuern des mobilen medizintechnischen Geräts ausgebildet ist; und
- eine Steuersignal-Schnittstelle (410), die zum Bereitstellen des bestimmten Steuersignals an eine Bewegungsaktorik des mobilen medizintechnischen Geräts ausgebildet ist.

13. Mobiles medizintechnisches Gerät umfassend einen Controller (400) gemäß dem direkt vorhergehenden Anspruch.

14. Computerprogrammprodukt mit Programmelementen, die eine Trainings-Vorrichtung (300) veranlassen, die Schritte des Verfahrens zum Trainieren eines dynamischen Kollisionsmodells für ein mobiles medizintechnisches Gerät gemäß einem der Ansprüche 1 bis 8 auszuführen, wenn die Programmelemente in einen Speicher der Trainings-Vorrichtung (300) geladen werden; und/oder mit Programmelementen, die einen Controller (400) veranlassen, die Schritte des Verfahrens zum Steuern einer Bewegung eines mobilen medizintechnischen Geräts anhand eines dynamischen Kollisionsmodells gemäß Anspruch 9 auszuführen, wenn die Programmelemente in den Speicher des Controllers (400) geladen werden.

15. Computerlesbares Medium, auf dem Programmelemente gespeichert sind, die von einer Trainings-Vorrichtung (300) gelesen und ausgeführt werden können, um Schritte des Verfahrens zum Trainieren eines dynamischen Kollisionsmodells für ein mobiles medizintechnisches Gerät gemäß einem der Ansprüche 1 bis 8 durchzuführen, wenn die Programmelemente von der Trainings-Vorrichtung (300) ausgeführt werden; und/oder auf dem Programmelemente gespeichert sind, die von einem Controller (400) gelesen und ausgeführt werden, um Schritte des Verfahrens zum Steuern einer Bewegung eines mobilen medizintechnischen Geräts anhand eines dynamischen Kollisionsmodells gemäß Anspruch 9 durchzuführen, wenn die Programmelemente von dem Controller (400) ausgeführt werden.
